# EUROPEAN PATENT SPECIFICATION

(11) **EP 4 098 176 B1**
(45) Date of publication and mention of the grant of the patent: **25.02.2026**
(21) Application number: 21177197.7
(22) Date of filing: 01.06.2021
(51) Int. Cl.: A61B 3/10

(54) **OPTICAL APPARATUS**
OPTISCHE VORRICHTUNG
APPAREIL OPTIQUE

(43) Date of publication of application: 07.12.2022
(73) Proprietor: Wavesense Engineering GmbH, 1190 Vienna (AT)
(72) Inventor: KUMAR, Abhishek, 1190 Wien (AT)
(74) Representative: Vossius & Partner Patentanwälte Rechtsanwälte mbB

(56) References cited:
- WO-A1-2014/053824
- WO-A1-2019/010507
- US-A1- 2018 020 912

## Description

The present disclosure relates to optical apparatuses, in particular optical apparatuses to calculate wavefront error at different planes in the eye.

The use of wavefront aberrometry for vision correction surgical procedures, such as custom LASIK (Laser-in-situ-Keratomileusis), and design of custom contact lenses and glasses is well established [1] [2]. Currently, there are several aberrometers based on different working principles available on the market with the most prominent being the Shack-Hartmann sensor [3] [4]. However, due to their limited dynamic range, they are mostly suitable for pre-operative examination.

The use of intra-operative aberrometry for guiding intra-ocular lens (IOL) implant surgery and surgical management of astigmatism have been demonstrated [5]. These are either based on Talbot interferometry or the time sequential waterfront sensing principle, which offer large diopter range [6] [7] suitable for measurement in an aphakic state, when the eye's optics becomes highly hyperopic.

Although wavefront aberrometry provides information regarding the optical properties of the eye, it lacks the biometry or anatomical information. Hence, intra-operative aberrometry measurements are still used with a generalized regression based IOL power calculation formula [8], thus limiting the accuracy of IOL power selection and subsequently the precision of the surgical outcome.

Both pre-operative and intra-operative optical coherence tomography (OCT) can provide anatomical and biometric information of the eye [9] [10] [11] [12] [13]. It has been shown that an IOL power calculation formula that uses anterior chamber depth (ACD) and real IOL position estimation yields more accurate results as compared to the modern new generation of regression-based formulae [14]. Although some optical properties such as corneal power and corneal astigmatism can be estimated using the anterior chamber OCT imaging, it has limited accuracy due to calibration issues, and subject's motion due to slow volume imaging speed [15] [16].

Since OCT can also provide phase information along with intensity based imaging, various digital and computational methods, termed as computational or digital adaptive optics (CAO or DAO), have been demonstrated that can compute the wavefront error in OCT images and also digitally correct it in post-processing [17] [18] [19] [20].

It is an object of the present invention to overcome the aforementioned deficiencies of the prior art and to provide apparatuses to calculate wavefront error at different planes in the eye. The object is achieved with the features of the independent claims. Dependent claims define preferred embodiments of the invention.

In particular, the present disclosure relates to an optical apparatus, comprising: a source of wavelength tunable laser light (100) or a broad band partially coherent light source (1001), wherein light from the wavelength tunable laser light (100) or the broad band partially coherent light source (1001) is split into a sample arm (110, 1010) as illumination light and a reference arm (120, 1020) as reference light, the sample arm (110, 1010) comprising: a first beam splitter (111, 1011), means for directing the illumination light via the first beam splitter (111, 1011) as a light spot to a sample, wherein an image of the light spot is reflected from the sample, a focus tunable optics (112, 1012) receiving the image of the light spot from the sample after being transmitted through the first beam splitter (111, 1011) and focusing the image to a detection plane, wherein a photodetector unit is adapted for receiving the recombined light from the sample arm (110, 1010) and the reference arm (120, 1020), wherein the sample arm (110, 1010) comprises a separate illumination channel configured to inject light via a mirror (116, 1003) and the first beam splitter (111, 1011) to the sample without passing through the focus tunable optics (112, 1012).

Various embodiments may preferably implement the following features.

Preferably, the focus tunable optics is configured to be controlled manually, wherein the focus tunable optics preferably comprises a plurality of lenses arranged in a Badal system, such that the image is focused to the detection plane.

Preferably, the focus tunable optics is configured to be controlled automatically, wherein the focus tunable optics preferably comprise electrically focus tunable liquid crystal optical elements, such that the image is focused to the detection plane.

Preferably, the focus tunable optics is adapted to increase the dynamic range of the signal detection more preferably adapted to compensate defocus and/or astigmatism of the sample.

Preferably, the sample is an eye and wherein the focus tunable optics is adapted to ensure that the retinal plane of the eye is continuously conjugated to or imaged at the detection plane.

Preferably, the optical apparatus further comprises a computing unit being connected to the photodetector unit, wherein the computing unit is configured for digitization and further data processing.

That is, the optical apparatus preferably further comprises a computing unit connected to the photodetector unit, wherein the computing unit is configured to digitize the signal and use digital techniques to calculate wavefront error at different planes, e.g. in the human eye.

Preferably, the sample arm further comprises a scanner, preferably a 2-D scanner placed at the Fourier plane of collimation optics located in front of the detection plane.

Preferably, the sample arm further comprises a detection fiber being adapted to receive the image of the illuminated spot at the detection plane and guide light to the photodetector and more preferably an actuator configured to translate the detection fiber laterally across the image of the illuminated spot.

Preferably, in an embodiment, the photodetector comprises of either a dual balanced photo detector or a spectrometer being adapted to receive the light reflect back from the illuminated spot at the detection plane, which is preferably conjugated to the retinal plane of the eye.

Preferably, in another embodiment, the photodetector comprises a 2-D camera sensor being adapted to receive the light reflect back from the illuminated spot at the detection plane, which is preferably conjugated to the pupil plane of the eye.

Preferably, the optical apparatus further comprises a first mirror directing the light from a broad band light source to the first beam splitter for splitting the light into the sample arm and the reference arm, wherein the reference arm further comprises: a second mirror adapted to receive light transmitted through the first beam splitter, a beam expander adapted to receive light via the second mirror and adapted to increase the light to a diameter of light preferably to a diameter of 5 to 10 mm, more preferably around 8 mm, a third mirror receiving the light from the beam expander and directing it to a diffraction grating for directing the reference light to a second beam splitter adapted to combine with the light reflected back from the sample arm passing through the first beam splitter and the focus tunable optics, and wherein the combined sample and reference light directed via the second beam splitter is preferably captured by the 2-D camera, which is preferably conjugated to the pupil plane of the eye.

Preferably, the computing unit is configured to generate a volume image of the sample preferably a full eye and point spread functions, PSF and to determine a wavefront error using a digital adaptive optics, DAO algorithm preferably a digital lateral shearing based digital adaptive optics algorithm, DLS-DAO algorithm.

According to the present disclosure, computational algorithm that can extract wavefront error information from digitized OCT data is considered as a DAO algorithm.

Preferably, the computing unit is configured to: obtain volumetric optical coherence tomography data, OCT data of PSF scans of the eye, extract an enface PSF field at a retinal layer of the eye after OCT based data processing, derive a defocus distance in an image space from a shift in the image plane at which an image of the light spot is best focused when the focal length of the focus tunable optics is changed, calculate 2-D fast Fourier transform, FFT of the PSF field and add a defocus phase corresponding to the derived defocused distance to the phase of the calculated Fourier field, numerically wave propagate the resulting field at Fourier plane to the image location of the pupil of the eye and reconstruct the phase or wavefront error using the digital adaptive optics, DAO algorithm, preferably the DSL-DAO algorithm from the calculated field at a pupil plane of the eye.

Preferably, the computing unit is configured to: obtain volumetric optical coherence tomography data, OCT data of PSF scans of the eye, extract an enface PSF field at a retinal layer of the eye after OCT based data processing, calculate 2-D FFT of the PSF field, numerically wave propagate the resulting field at Fourier plane to the image location of the pupil of the eye, derive a defocus distance of a focal plane of the eye from the retina, from a change in focus of the focus tunable optics, add a defocus phase corresponding to the derived defocused distance to the phase of the calculated pupil field, and reconstruct the phase or wavefront error using the digital adaptive optics, DAO algorithm, preferably the DSL-DAO algorithm from the calculated field at a pupil plane of the eye.

Preferably, the computing unit is configured to: obtain volumetric optical coherence tomography data, OCT data of PSF scans of the eye in an aphakic state, extract an enface PSF field at a retinal layer after OCT based data processing, derive a defocus distance of a focal plane of the aphakic eye from the retina, from a change in focus of the focus tunable optics, numerically wave propagate the PSF field to an estimated IOL location plane within the eye taking into account the defocus distance, and reconstruct the phase or wavefront error using the digital adaptive optics, DAO algorithm, preferably the DSL-DAO algorithm from the calculated field at the IOL location plane within the eye.

Preferably, the computing unit is configured to evaluate an IOL performance preferably comprising: multiplying a field at the IOL location plane with a complex exponential of a known phase of a selected IOL, calculating the field back at the retinal plane using a numerical wave propagation algorithm, and calculating a spot size and a modulus transfer function, MTF for quantifying a visual performance.

Preferably, the computing unit is configured to: obtain volumetric optical coherence tomography data, OCT data of PSF scans of the eye in a phakic state, extract an enface PSF field at a retinal layer after OCT based data processing, derive a defocus distance of a focal plane of the phakic eye from the retina, from a change in focus of the focus tunable optics, numerically wave propagate the PSF field to a plane at a last surface of a crystalline lens considering vitreous media within the eye and taking account a defocus distance, multiply the calculated field with a complex conjugate of a transmission function of the crystalline lens to cancel its refractive effect, numerically wave propagate the resulting field to an estimated IOL location, and reconstruct the phase or wavefront error using the digital adaptive optics, DAO algorithm, preferably the DSL-DAO algorithm from the calculated field at an IOL location plane within the eye.

Preferably, the computing unit is configured to determine at least one of a sphere, a cylinder and cylinder axis values from the reconstructed phase error and to use the determined values for selecting an IOL or for designing a custom IOL having an optimal sphere and cylinder power and cylinder axis for correcting the phase or wavefront error of the eye.

Preferably, the computing unit is configured to evaluate an IOL performance preferably comprising: multiplying a field at the IOL location plane with the transmission function of a selected IOL, calculating the field back at the retinal plane using a numerical wave propagation algorithm and calculating a spot size and a modulus transfer function, MTF for quantifying a visual performance.

Preferably, the computing unit is configured to: numerically wave propagate a sample field at a camera plane to the image location of the pupil of the eye, derive a defocus phase at a pupil plane of the eye corresponding to a focal length change of the focus tunable optics, add the derived defocus phase to the phase of a calculated pupil field, and reconstruct a phase or wavefront error using the digital adaptive optics, DAO algorithm, preferably the DLS-DAO algorithm from the calculated field at the pupil plane of the eye.

Preferably, the computing unit is configured to: provide PSF corresponding to the retinal layer and wavefront error of an eye, determine cylinder power and axis of an astigmatism or cylinder error correcting toric IOL, determine residual cylinder error after toric IOL is implanted in an eye during the IOL implant surgery from a quality metric based on spot size of the PSF profile, determine angle by which toric IOL needs to be rotated to achieve optimal axis alignment in order to cancel the residual cylinder error, and finally confirm the accuracy of the axis alignment of the toric IOL based on a quality metric based on spot size of the PSF profile.

The embodiments aim at utilizing the combination of full eye OCT imaging and wavefront aberrometry based on DAO in order to get full information regarding the optical and biometric property of the whole eye, which can then be used for more accurate IOL power calculations, for evaluation of various IOL designs and also help guide accurate IOL positioning.

Furthermore, an embodiment for digital aberrometry is based on an off-axis digital holography approach that can be useful in situations where only wavefront error measurement is required without biometry.

Embodiments of the present disclosure may allow wavefront error calculation for a human eye suitable for clinical applications with high accuracy over a wide dynamic range.

The exemplary embodiments disclosed herein are directed to providing features that will become readily apparent by reference to the following description when taken in conjunction with the accompany drawings. In accordance with various embodiments, exemplary systems, methods, devices and computer program products are disclosed herein. It is understood, however, that these embodiments are presented by way of example and not limitation, and it will be apparent to those of ordinary skill in the art who read the present disclosure that various modifications to the disclosed embodiments can be made while remaining within the scope of the present disclosure.

Thus, the present disclosure is not limited to the exemplary embodiments and applications described and illustrated herein. Additionally, the specific order and/or hierarchy of steps in the methods disclosed herein are merely exemplary approaches. Based upon design preferences, the specific order or hierarchy of steps of the disclosed methods or processes can be re-arranged while remaining within the scope of the present disclosure. Thus, those of ordinary skill in the art will understand that the methods and techniques disclosed herein present various steps or acts in a sample order, and the present disclosure is not limited to the specific order or hierarchy presented unless expressly stated otherwise.

The above and other aspects and their implementations are described in greater detail in the drawings, the descriptions, and the claims.
FIG. 1 shows a optical coherence tomography system according to an embodiment.
FIG. 2 is a schematic drawing of the sample arm according to an embodiment.
FIG. 3 is a schematic drawing of the sample arm according to an embodiment.
FIG. 4a shows a flow chart according to an embodiment.
FIG. 4b shows a flow chart according to an embodiment.
FIG. 5 shows a schematic drawing of an eye and a position of an IOL.
FIG. 6 shows a flowchart of an embodiment.
FIG. 7 shows a flowchart of an embodiment.
FIG. 8 shows a multimodal optical coherence tomography system according to an embodiment.
FIG. 9 shows schematic drawings according to embodiments.
FIG. 10(a) shows a schematic illustration of a wavefront sensor based on digital off-axis holography according to an embodiment.
FIG. 10(b) shows a flowchart of post-processing steps to extract the sample field at the camera detection plane according to an embodiment.
FIG. 11 shows a flowchart of the post processing steps to calculate wavefront error of the eye from the sample field obtained using the off-axis digital holography scheme according to an embodiment.

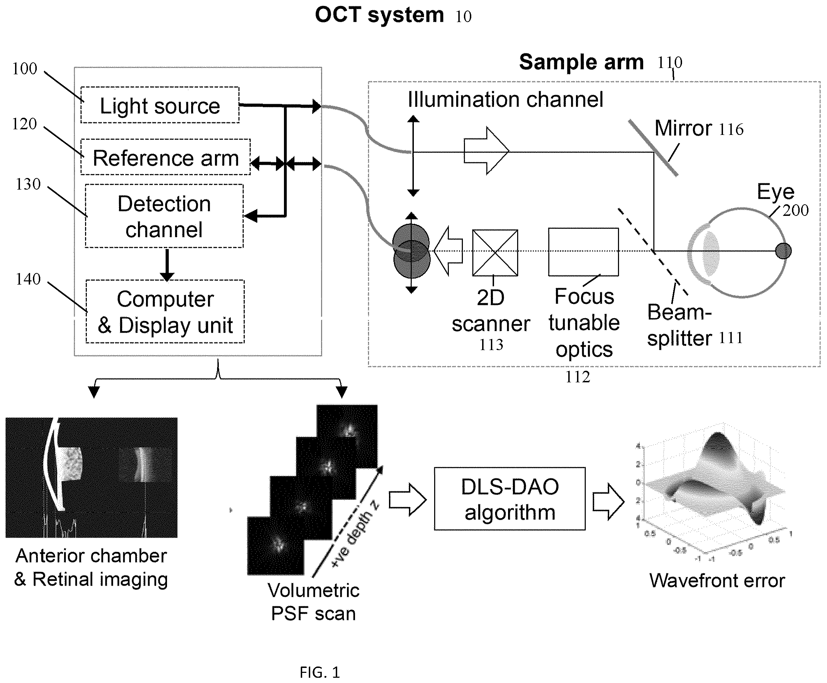

FIG. 1 shows a optical coherence tomography (OCT) system 10 according to an embodiment of the present disclsoure. The OCT system 10 comprises a light source 100, a sample arm 110, a reference arm 120, a detection channel 130, a computer and display unit 140. The sample arm 110 comprises an illumination channel with a mirror 116, a beam splitter 111, focus tunable optics 112 and a 2-dimensional (2D) scanner 113. FIG. 1 further shows an eye 200 of a patient. The thick arrows in front of the mirror 116 and behind the 2D scanner 113 indicate the direction of the propagation of the light beam.

In general, the multimodal OCT system 10 of FIG. 1 can generate both a volume image of the full eye 200 and the PSF, which on processing with the DLS-DAO algorithm on a computer yields the wavefront error. Further details and components of the OCT system 10 are described in the following.

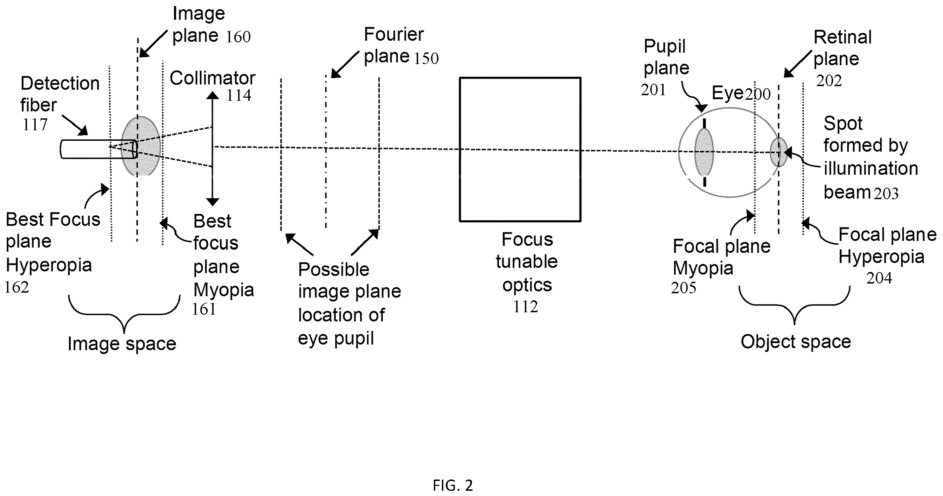

FIG. 2 a schematic drawing of the sample arm 110 showing the eye 200, the pupil plane 201, the retinal plane 202, the spot 203 formed by an illumination beam, the focal plane Hyperopia 204, and the focal plane Myopia. FIG. 2 further shows the focus tunable optics 112, the Fourier plane 150, a collimator 114, a detection fiber 117, an image plane 160, a best focus plane Myopia 161, and a best focus plane Hyperopia 162.

In general, FIG. 2 shows a schematic drawing of the sample arm 110 showing different planes with respect to the whole optical setup, which includes the eye 200 in the object space, focus tunable optics 112 that focus the image of the illumination spot on the tip of the detection fiber 117 placed after the collimator 114, which is in the image space. Illumination channel and scanner are not shown in FIG. 2 in order to simplify the diagram.

With reference to FIGs. 1 and 2, embodiments of the present disclosure are described.

As can be seen in FIG. 1, an optical apparatus (OCT system 10) is provided to calculate wavefront error at different planes in the eye in different conditions such as 1) phakia, 2) aphakia and 3) pseudophakia using a multimodal ophthalmic OCT system 10 that can deliver volumetric PSF (point spread function) scan of the eye with both amplitude and phase information, which can be processed using digital or computational adaptive optics techniques in particular digital lateral shearing based digital adaptive optics (DLS-DAO).

In FIG. 1, the light from the light source 100, which can either be a swept source laser or a broadband laser, is split into the sample and the reference arm 110, 120. The light reflected back from the sample 110 and the reference arm 120 is combined and detected by either a dual balanced photo-detector or spectrometer based detector.

The data is digitized and processed on a computer and displayed via a display unit (computer and display unit 140). A double path channel, which consists of 2-D scanner 113 and focus tunable optics 112, is used to deliver light to and from the eye 200 for imaging.

In order to scan the PSF, the sample arm 110 is adapted to include a separate illumination channel that can inject a narrow beam of light of diameter of less than 1 mm in the eye 200 via the mirror 116 and the beam splitter 111, so that a perfect diffraction limited spot could be formed on the retina.

The image of the spot is translated over the single mode detection fiber 117 in the sample arm 110. The method of PSF scan with an OCT based system is described in more detail in [20].

According to the embodiment, a focus tunable optic 112 is included in the sample arm110 to increase the dynamic range of the signal detection. This focus tunable optics 112, which may consist of plurality of lenses with one or more focus tunable optical element, can be adapted to compensate both defocus and astigmatism. Either manual or electrically focus tunable optical element(s) can be used. However, electrically focus tunable liquid crystal optical elements are preferred for real time applications where fast focus tuning is required.

The lenses in the tunable optics 112 can also be arranged as in a Badal system in case mechanical focus tuning is preferred over fast automatic focus tuning. With the focus tunable system, it is ensured that the retinal plane is always conjugated to or imaged at the detection fiber plane. Note that without the focus tunable optics 112, in the presence of defocus error due to myopia or hyperopia, the best focus plane for the illumination spot could be shifted away from the detection fiber plane as shown in FIG. 2. Thus, a blurred spot will be formed at the detection fiber 117, which will result in reduced irradiance and loss of signal coupling.

With a defocus error of > ± 2 diopters, the signal-to-noise ratio (SNR) can even drop to a level that the PSF cannot be recorded. Thus, focus tunable optics 112 are preferably be used in order to form a focused image of the illuminated spot on retina and keep the SNR of the detected PSF signal high. This can allow the scanned volumetric PSF detection using an OCT system 10 in the defocus error range of > ± 20 diopters, which can make the system versatile and robust in different scenarios.

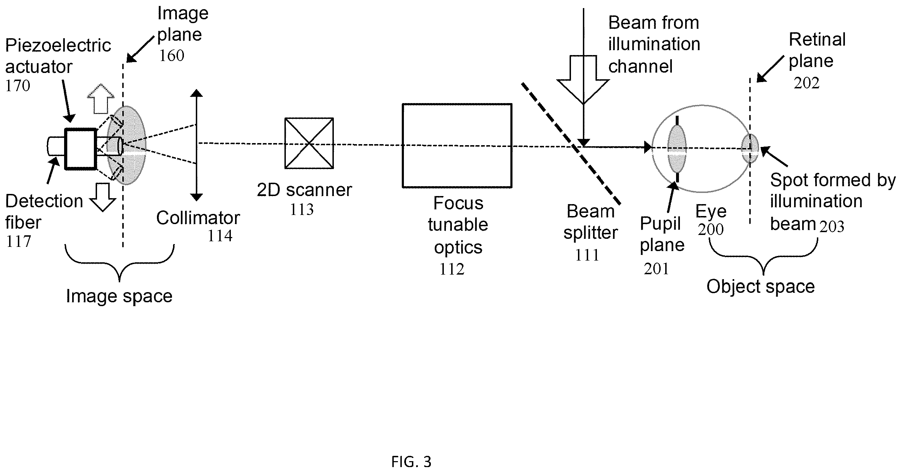

FIG. 3 shows an embodiment of the sample arm 110 showing the eye 200, the pupil plane 201, the retinal plane 202, the spot 203 formed by the illumination beam, the beam splitter 111, the focus tunable optics 112, the 2D scanner 113, the collimator 114, the image plane 160, the detection fiber 117, and a piezoelectric actuator 170.

In general, FIG. 3 shows the sample arm 110, wherein the piezo-electric actuator 170 is used to laterally translate the detection fiber tip across the image of the illumination spot 203. During the piezo-electric actuator operation, the scanner 113 is held stationary so that image of the illumination spot 203 is also stationary.

FIG. 3 shows the sample arm 110 wherein an actuator 170 (e.g. a piezoelectric actuator) is attached to the detection fiber 117. The transducer translates the detection fiber 117 laterally across the image of the illuminated spot 203, which is equivalently the PSF of the eye 200. In this case the 2-D scanner 113 of the sample arm 110 is held stationary so that the image of the illumination spot 203 is also stationary while the detection fiber 117 is translated, which also results in the lateral sampling of the PSF profile.

Control of the focus tunable optics 112 system is done through a calibration process. A physical model eye that can mimic a normal and a defocused eye can be used. Defocus error in the physical model eye can be introduced either by adding a sphere lens with varying diopters in the pupil plane or by changing the axis length.

With the physical model eye set to a normal condition of zero diopter defocus error, a 2D PSF profile corresponding to the retinal layer is obtained with the modified OCT system as illustrated in FIG. 1. A circle with encircled energy of around 86% of total energy of the PSF profile is determined and its diameter value is then used as an ideal case value for a focused spot size.

In case of electrical focus tunable optics system, in which liquid crystal based optical elements with application of different voltage change curvature and thus focus, voltage can be tuned to a value that provides the focused PSF spot with a diameter that matches the determined ideal case value. Defocus error of the physical model eye can be progressively changed from -20 diopters to +20 diopters in a step of 0.25 diopters, and for each defocus error condition within that range a corresponding voltage for the focus tunable system 112 that can provide the ideal focused PSF spot size can be determined.

In case of mechanical focus tuning based on a Badal system, in which a distance between the two lenses in the Badal arrangement is varied for example by moving one of the lenses on a translation stage in order to change the focus of the system, a corresponding distance between the Badal system lenses that provides the focused PSF spot with diameter that matches with the determined ideal case value can be determined for a given defocus condition of the physical model eye.

Even though defocus and possibly astigmatism can roughly be compensated with the focus tunable system 112 in order to enhance signal detection in the wide range of defocus error of ± > 20 diopters, there can still be residual sphere and cylinder error of < 1 diopters left along with significant higher order aberrations (HOA). These aberrations can be detected using a computational or digital adaptive optics technique, in particular a DLS-DAO technique.

In order to be clinically preferable for vision correction, the wavefront error or aberration may be calculated at 1) the pupil plane 201 in cases where corneal treatment (e.g. LASIK), contact lens and custom glasses are required; 2) the estimated position of intra ocular lens (IOL) implant in cases where IOL implant is necessary (e.g. cataract surgery).

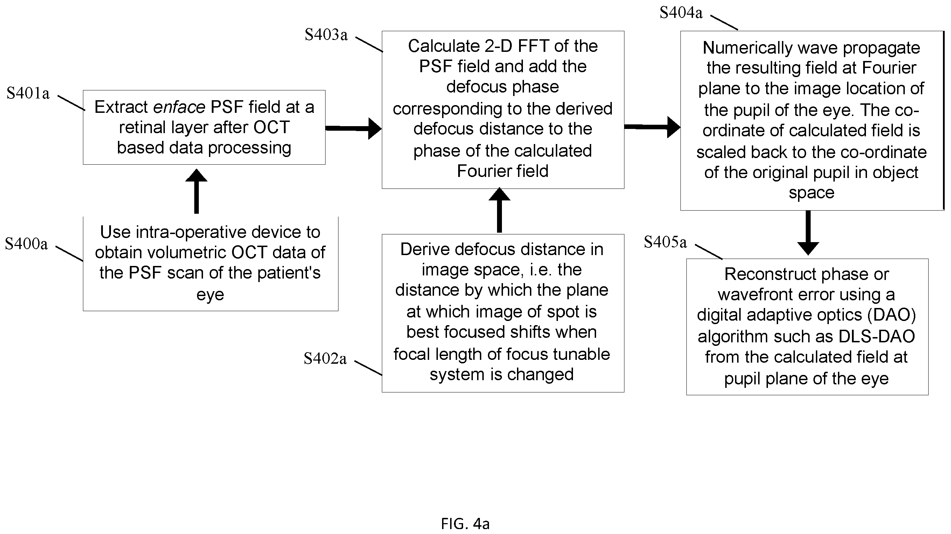

FIG. 4a shows a flow chart according to an embodiment of the disclosure. In general, FIG. 4a shows steps used in the process to calculate the optical field and the wavefront error at the pupil plane 201. The process is applicable to an eye 200 in a phakic, aphakic and pseudophakic state.

In particular, at step S400a, the intra-operative device is used to obtain volumetric OCT data of the PSF scan of the patient's eye 200. At step 401a the enface PSF field is extracted at a retinal layer after OCT based data processing. At step S402a the defocus distance in the image space is derived, i.e. the distance by which the plane at which the image of the spot is best focused shifts when the focal length of the focus tunable system is changed.

At S403a the 2D-FFT of the PSF field is calculated and the defocus phase corresponding to the derived defocus distance is added to the phase of the calculated Fourier field. At S404a the resulting field at the Fourier plane is numerically wave propagated to the image location of the pupil of the eye. The coordinate of the calculated field is scaled back to the co-ordinate of the original pupil in the object space. At S405a the phase or wavefront error is reconstructed using digital adaptive optics (DAO) algorithm such as DLS-DAO from the calculated field at the pupil plane of the eye.

In particular, in the computation or digital adaptive optics approach, when a 2-D FFT of the selected enface PSF field from the volumetric scanned PSF data is calculated on a computer, it yields the field information at the Fourier plane of the collimation lens 114 placed just in front of the detection fiber 117.

The phase of the calculated Fourier field may be adjusted in case a focus tunable lens system 112 is used in the detection path to compensate the defocus or the spherical equivalent error of the eye 200 and keep the retinal plane 202 conjugated to the detection fiber plane. In this case, a relationship between the defocus distance in the image space and change in focal length of the focus tunable lens system 112, from the set ideal focal length for the case of normal emmetropic eye, is established using a theoretical modeling of the system using either 1) an approximate ABCD matrix formulation or 2) computer simulation of the system with a ray tracing software (eg. Zemax, Code V etc.).

The defocus phase error corresponding to defocus distance in the image space is calculated. The calculated Fourier field is pixel-by-pixel multiplied by a complex exponential function with unit amplitude and argument as the calculated defocus phase, as shown in FIG. 4a. The Fourier plane may not coincide with the image plane of the pupil formed by the focus tunable lens arrangement 112.

The image location of the pupil for different focus configurations of the focus tunable lens system 112 can be determined by using 1) approximate ABCD matrix formulation or 2) precisely with a ray tracing software. Using either method a lookup table of the distance between the Fourier and the image of the pupil plane, for different focus configuration of the focus tunable lens system 112, can be determined.

Using the known distance, the calculated Fourier field can be numerically wave propagated on the computer to the location of the image of the pupil using a numerical technique such as Angular spectrum or Fresnel propagation or a combination or a variation of both, which yields the field at the image location of the pupil plane 201.

Finally, the coordinate of the image of the pupil plane can be scaled back to the original pupil plane dimension by taking into account the magnification factor between the image and the original pupil plane. This approach is suitable for the pupil plane field calculation irrespective of whether the eye is in phakic, aphakic or pseudo-phakic state. Once the pupil field is determined, wavefront error at the pupil plane can be calculated using a digital adaptive optics technique such as DLS-DAO. The flow chart of the respective method is shown in FIG. 4a.

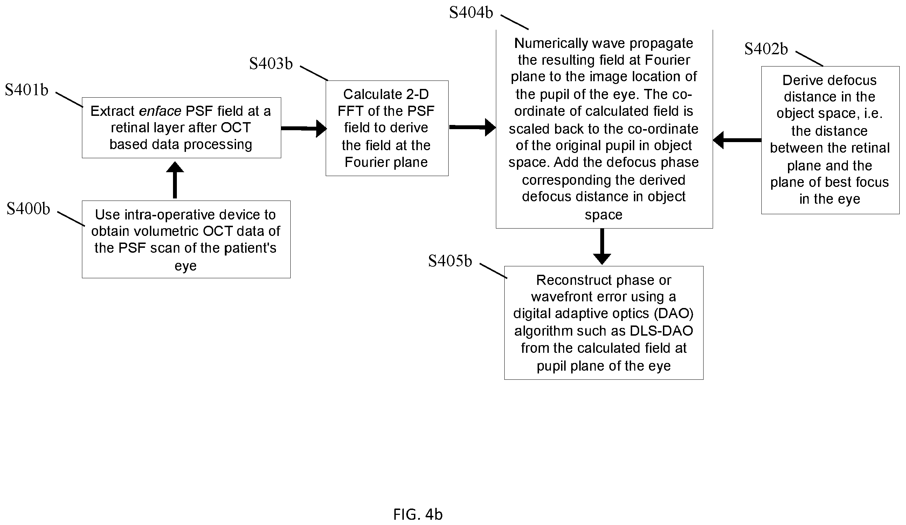

An alternative way to take into account the defocus phase due to change in the focal length of the focus tunable lens system 112 is shown in Fig. 4b. In this case, the Fourier field of the extracted *enface* PSF field is directly numerically wave propagated to the image location of the pupil plane and the coordinates scaled back according to the coordinates of the object space. The derived defocus phase corresponding to the defocus distance in the object space, i.e. the distance between the retinal plane and the plane of best focus of the eye 200 based on the numerical modeling of the physical eye 200, is then added to the phase of this calculated pupil field. Finally, the net pupil field is processed using a digital adaptive optics technique, such as DLS-DAO, in order to calculate the wavefront error of the eye 200 at the pupil plane 201.

FIG. 4b shows a flow chart according to an embodiment of the disclosure. In general, FIG. 4b shows steps used in the process to calculate the optical field and the wavefront error at the pupil plane 201. The process is applicable to an eye 200 in a phakic, aphakic and pseudophakic state.

In particular, at step S400b, the intra-operative device is used to obtain volumetric OCT data of the PSF scan of the patient's eye 200. At step 401b the enface PSF field is extracted at a retinal layer after OCT based data processing. At step S402b the defocus distance in the object space is derived, i.e. the distance between the retinal plane and the plane of the best focus in the eye.

At S403b the 2D-FFT of the PSF field is calculated to derive the field at the Fourier plane. At S404b the resulting field at the Fourier plane is numerically wave propagated to the image location of the pupil of the eye. The coordinate of the calculated field is scaled back to the coordinate of the original pupil in the object space. The defocuse phase corresponding to the derived defocus distance in the object space is added. At S405b the phase or wavefront error is reconstructed using digital adaptive optics (DAO) algorithm such as DLS-DAO from the calculated field at the pupil plane of the eye.

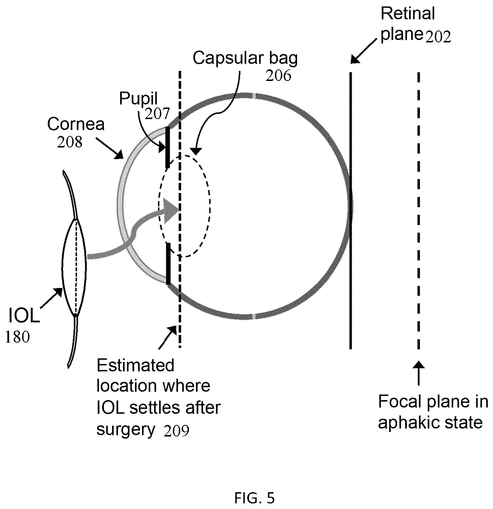

FIG. 5 shows a schematic drawing of the eye 200, the retinal plane 202, the capsular bag 206, the pupil 207, the cornea 208, and an intraocular lens (IOL) 180. In the aphakic state, when the natural crystalline lens of the eye 200 is removed, the eye 200 becomes hyperopic and the focal plane of the eye 200 shifts behind the retina. In cataract surgery IOL 180 is inserted in the capsular bag 206, where it settles in a final position.

The calculation of the field at the estimated location 209 of IOL 180, as shown in FIG. 5, during cataract surgery may help in the evaluation, selection and customized manufacturing of the IOL 180 with suitable power and design that can provide the best visual outcome for the patient. The estimation of the location where IOL 180 will settle after the implant surgery can be obtained using the anterior segment OCT image of the eye [21] [10] [9].

According to the disclosure, a PSF field in the image space is first scaled to the co-ordinate in the object space taking into account the magnification of the optical system, which effectively yields the virtual PSF field at retina, which by reversibility principle in optics is equivalent to the focused light field distribution at the retina due to the incoming light beam passing through the full pupil 207 and refracting through various refractive surfaces and optical media of the eye 200.

In case the measurement is done in the aphakic state during the surgery, the virtual PSF field at the retina can be numerically wave propagated, taking into account refractive index of vitreous media to an estimated IOL location 209 directly.

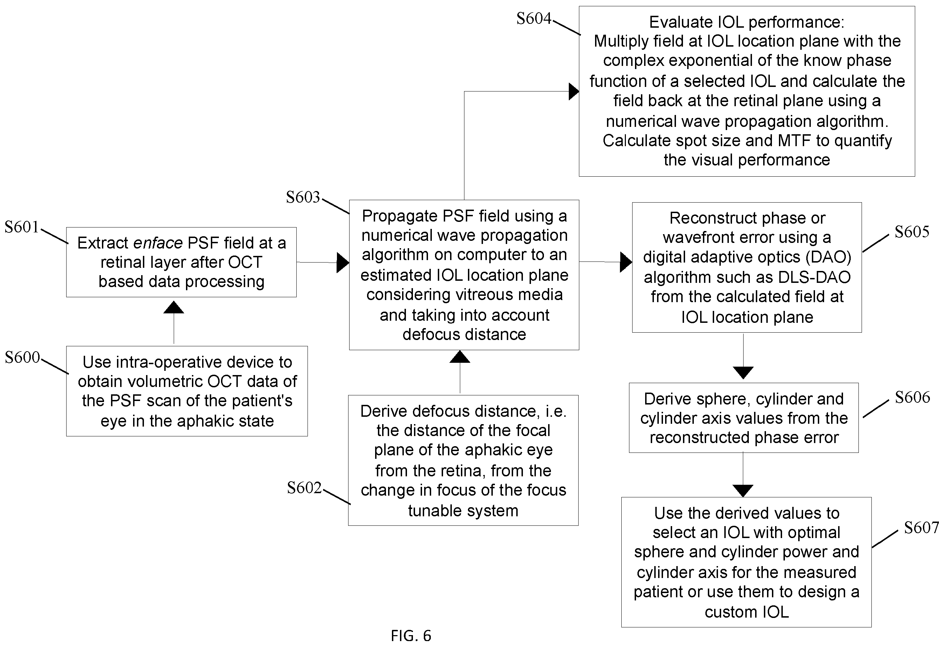

In case the defocus error of the eye 200 is compensated using a focus tunable system 112, the defocus distance in the object space corresponding to the change in focal length of the focus tunable system 112 is calculated, which is then added to the propagation distance, while propagating the PSF field to the estimated location 209 of the IOL 180. The flow chart of the method is shown in FIG. 6.

FIG. 6 shows a flowchart of an embodiment of the present disclosure. In general, FIG. 6 shows steps used in the process to calculate the optical field and the wavefront error at the estimated IOL location 209 for an eye in aphakic state, which can be further used to design and evaluate the optical and visual performance of the IOL.

In particular, at step S600, the intra-operative device is used to obtain volumetric OCT data of the PSF scan of the patient's eye 200 in the aphakic state. At step S601 the enface PSF field is extracted at a retinal layer after OCT based data processing. At step S602 the defocus distance is derived, i.e. the distance of the focal plane of the aphakic eye from the retina, from the change of focus of the focus tunable system.

At S603 the resulting PSF field propagated using a numerically wave propagation algorithm on a computer (processor) to an estimated IOL location plane considering vitreous media and taking the defocus distance into account. At S604 the IOL performance is evaluated by multiplying the field at the IOL location plane with the complex exponential of the known phase function of a selected IOL, calculating the field back at the retinal plane using a numerical wave propagation algorithm and calculating the spot size and MTF to quantify the visual performance.

At S605 the phase or wavefront error is reconstructed using a digital adaptive optics (DAO) algorithm such as DLS-DAO from the calculated field at the IOL location plane. At S606 the sphere, cylinder and cylinder axis values are derived from the reconstructed phase error. At S607 the derived values are used to select an IOL with optimal sphere and cylinder power and cylinder axis for the measured patient or the derived values are used to design a custom IOL.

In case of phakic eye, the virtual PSF field at the retina plane 202 can be numerically propagated through the vitreous media to the back surface of the crystalline lens taking into account the distance between the last surface of the crystalline lens and retina and the refractive index of the vitreous media.

In case the defocus error of the eye 200 is compensated using a focus tunable system 112, the defocus distance in the object space corresponding to the change in focal length of the focus tunable system 112 is calculated, which is then added to the propagation distance while propagating the PSF field to the last surface of the crystalline lens.

If the crystalline lens front and rear surface curvature, thickness and effective refractive index can be determined from 2-D or 3-D anatomical OCT imaging, then the phase function of the crystalline lens can be derived and the corresponding phase transmission function, which is the complex exponential function with unit amplitude and argument as lens phase function, can be determined.

Since the phase of the wavefront is most relevant, an assumption of unit amplitude may be sufficient. The calculated field at the back surface of the crystalline lens can be multiplied pixel-by-pixel with the complex conjugate of the determined lens phase transmission function, which cancels out the refraction effect of the lens and yields the field at the front surface on the crystalline lens.

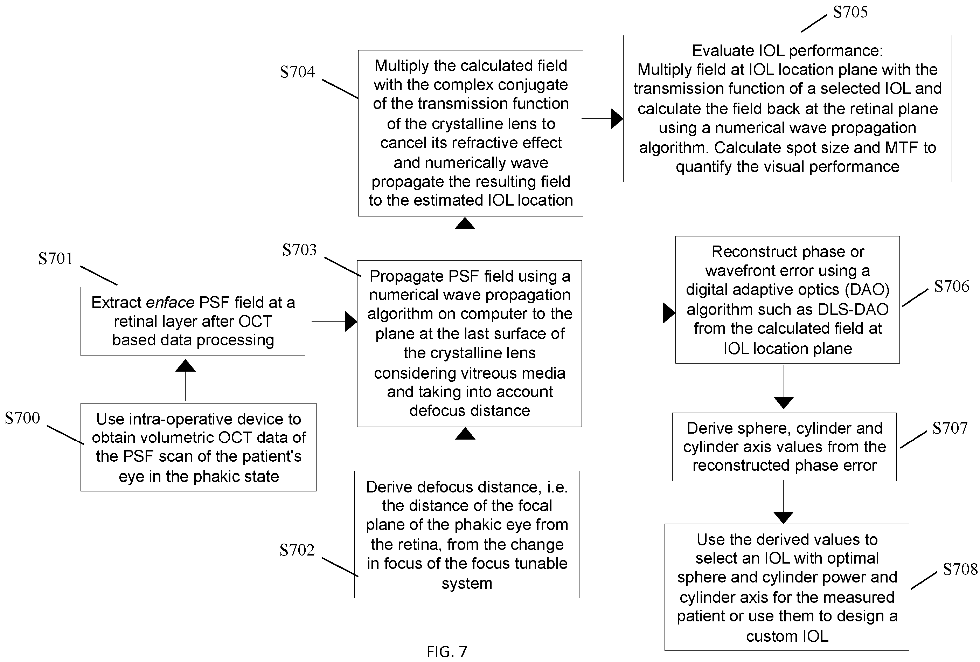

The calculated field at the front surface of the crystalline lens is effectively equivalent to the field due to the planar wavefront refracting through anterior and posterior cornea, passing though the full pupil and propagating through the aqueous humour media to the front lens surface. The flow chart of the method is shown in FIG. 7.

FIG. 7 shows a flowchart of an embodiment of the present disclosure. In general, FIG. 7 shows steps used in the process to calculate the optical field and the wavefront error at the estimated IOL location for an eye in phakic state before an IOL implantation, which can be further used to design and evaluate the optical and visual performance of the IOL 180.

In particular, at step S700, the intra-operative device is used to obtain volumetric OCT data of the PSF scan of the patient's eye 200 in the phakic state. At step S701 the enface PSF field is extracted at a retinal layer after OCT based data processing. At step S702 the defocus distance is derived, i.e. the distance of the focal plane of the phakic eye from the retina, from the change in focus of the focus tunable system.

At S703 the PSF field is propagated using a numerically wave propagation algorithm on a computer (processor) to the plane at the last surface of the crystalline lens considering vitreous media and taking the defocus distance into account. At S704 the calculated field is multiplied with the complex conjugate of the transmission function of the crystalline lens to cancel its refractive effect and numerically wave propagate the resulting field to the estimated IOL location.

At S705 the IOL performance is evaluated by multiplying the field at the IOL location plane with the transmission function of a selected IOL, calculating the field back at the retinal plane using a numerical wave propagation algorithm and calculating the spot size and MTF to quantify the visual performance.

At S706 the phase or wavefront error is reconstructed using a digital adaptive optics (DAO) algorithm such as DLS-DAO from the calculated field at the IOL location plane. At S707 the sphere, cylinder and cylinder axis values are derived from the reconstructed phase error. At S708 the derived values are used to select an IOL with optimal sphere and cylinder power and cylinder axis for the measured patient or the derived values are used to design a custom IOL.

A computational or DAO technique, in particular DLS-DAO, can be applied to the calculated field at the IOL location in either the phakic or aphakic case in order to determine the wavefront error or deviation from spherical wavefront with radius of curvature equal to the distance of the IOL location from fovea of the retina as shown in FIGs. 6 and 7.

Based on the determined wavefront error, sphere and cylinder power and cylinder axis of the IOL 180 can be determined that results in an optimal focused spot on the retina free from sphere and cylinder error that can ensure 20/20 vision for the patient.

Also, based on the determined wavefront error, a custom IOL 180 can be designed and manufactured with anterior and posterior refracting surfaces, thickness and refractive index that can not only minimize second order aberration such as sphere and cylinder but also higher order aberrations (HOAs) such as spherical, coma, trefoil etc. to ensure best visual performance for the patient.

Furthermore, a simulation of objective refraction can be done to analyze the visual performance of the designed IOL or an IOL with selected sphere and cylinder power and cylinder axis. In this case, the calculated field at the location of the front surface of IOL is pixel-by-pixel multiplied by the phase transmission function of the IOL, and then numerically wave propagated in vitreous media to the retinal plane 202 to get the focused spot. The root mean square (RMS) spot size and the corresponding modulus transfer function (MTF) are quantified for visual performance analysis.

The steps shown in the flowchart of FIG. 4a, FIG. 4b, FIG. 6, and FIG. 7 are not restricted to intra-operative devices, but are also valid and can be used if OCT volume data of the PSF scan is avaible via a pre-operative or post-operative device.

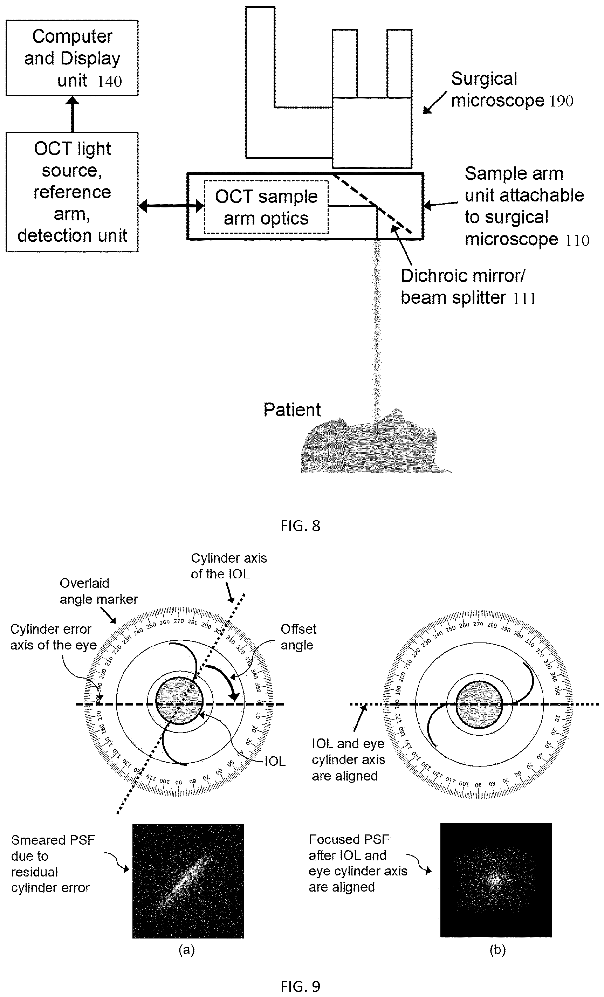

FIG. 8 shows an embodiment of the multimodal OCT system, which is adapted such that it can be attached to a surgical microscope 190 for use during a surgery. In particular, FIG. 8 shows a sample arm unit 110 attachable to the surgical microscope 190, an OCT light source, reference arm and detection unit and a computer and display unit 140, wherein a dichroic mirror/beam splitter 111 and OCT sample arm optics are located within the sample arm unit 110.

FIG. 9 shows schematic drawings according to embodiments of the present disclosure, showing how a PSF imaging can guide IOL alignment. FIG. 9(a) shows a situation when an axis of the IOL and a cylinder error axis of the eye are misaligned by an offset angle resulting in a smeared PSF. FIG.9(b) shows a situation when a perfect alignment is achieved, which results in a tightly focused PSF.

That is, FIG. 8 shows an embodiment of a multimodal OCT system 10 described in FIG. 1. In this case the sample arm 110 is adapted such that it can be mounted or attached to the surgical microscope 190. A dichroic mirror 111 is used that can direct the near infrared illumination light from the sample arm 110 to the patient's eye and the reflected light back to the sample arm 110 without interrupting the original optical path and the view of the surgical microscope 190.

According to the disclosure, the scanned PSF obtained using an OCT system 10 can also be used to guide IOL implant surgery, which may be particularly useful in guiding the alignment of the astigmatism or cylinder error correcting toric IOL.

When the axis of the toric IOL is not aligned with the measured cylinder error axis of the eye, a smeared PSF will be obtained using the PSF generating OCT system, as shown in FIG. 9a. The surgeon can keep rotating the toric IOL, i.e. clockwise or anti-clock wise, until the position where the cylinder axis is cancelled and a reasonably focused PSF, wherein measured width of the PSF profile is within a predetermined set criterion that can ensure 20/20 visual performance, is obtained as shown in FIG. 9b. The surgeon may be guided in the right direction of rotation based on the criteria that the smearing or the width of the PSF profile has reduced or not by the direction of rotation from the initial starting point of measurement. The reduction of smearing and PSF width will indicate that the rotation direction is correct.

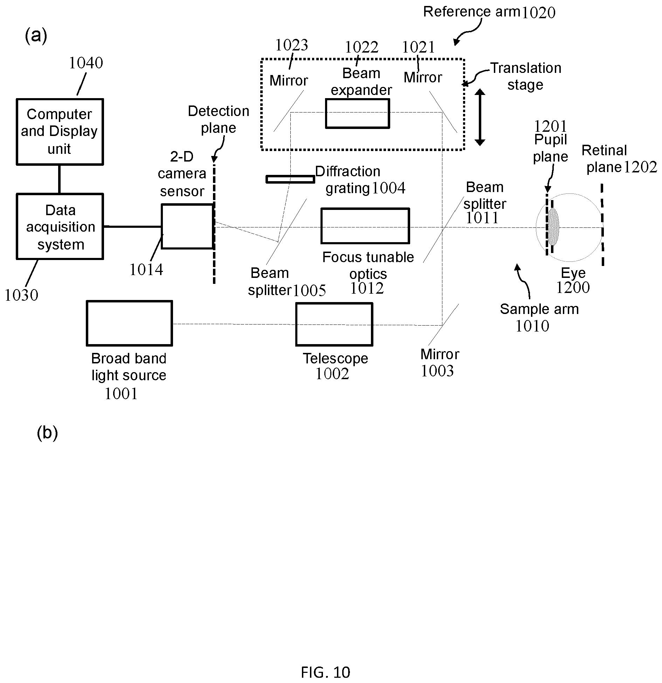

FIG. 10(a) shows schematic illustration of the wavefront sensor based on digital off-axis holography according to an embodiment of the present disclosure. In particular, FIG. 10(a) shows a broadband light source 1001, a telescope 1002, a mirror 1003, a beam splitter 1011, focuse tunable optics 1012, a beam splitter 1005, a diffraction grating 1004, a sample arm 1010, a 2D camera sensor 1014, a data acquisition system 1030, and a computer and display unit 1040. FIG. 10(a) further shows a reference arm 1020 with a mirror 1021, a beam expander 1022 and another mirror 1023. FIG. 10(a) also shows an eye 1200, a pupil plane 1201, and a retinal plane 1202.

FIG. 10(b) shows a flowchart of post-processing steps to extract the sample field at the camera detection plane, wherein the hologram/2D camera plane is subject to 2D FFT. From the conjugate of the object term in Fourier plane the object term is filtered out and subjected to 2D inverse fast Fourier transform, IFFT to obtain the sample field at the camera detection plane.

While considering the need for only standalone aberrometry, i.e. without biometry, a setup similar to full field off-axis digital holography may be useful that can exploit the advantages of digital wavefront error calculation while still being economically attractive.

The schematic of the setup is shown in FIG. 10(a). A collimated beam of light from a broad band light source 1001 is first reduced in beam diameter size to around 0.6 to 0.7 mm using a telescope 1002 and directed to a beam splitter 1011 using a mirror 1003, where the light is split into the sample and the reference arm 1010, 1020.

The light in the sample arm 1010 is injected into the eye 1200 and is focused on the retina to form an ideal spot, which acts as a pseudo point source of light reflecting light back. The light that is reflected back from retina is passed through the full aperture of the eye's pupil of diameter size between 3 to 7 mm, and is transmitted through the beam splitter 1011 and focus tunable optics 1012, which may consist of plurality of lenses in which one or more optical elements can change their focal length using either an electrical or mechanical mechanism.

The lenses in the tunable optics system 1012 can also be arranged as in a Badal system. The focus tunable optics 1012 is used for rough compensation of defocus and optionally cylinder error of the eye 1200 in order to increase the dynamic range of wavefront error detection.

The light passing through the tunable optics 1012 is transmitted through a beam splitter 1005 and is captured by a 2-D camera sensor 1014, where it is interfered with the light from the reference arm 1020. The detection plane of the camera sensor 1014 is placed at the focal plane of the tunable optics system 1012.

The light that is transmitted by the beam splitter 1011 placed just before the eye 1200 is directed to a beam expander 1022, whereby the beam diameter is increased to around 8 mm.

The expanded light is then directed to a diffraction grating 1004 via a mirror 1023. The arrangement of beam expander 1022 and mirrors 1021 and 1023 can be mounted on a translation stage that can change the path length in the reference arm 1020 in order to match the path length in the sample arm 1010 and control the coherence gate.

The diffracted light of first order from the diffraction grating 1004 is then reflected by the beam splitter 1005 to camera 1014 where it interferes with the light from the sample. The generated interference pattern or the hologram is captured by the camera 1014 and the data acquisition system 1030 which is then further processed on the computer and display unit 1040 in order to extract the field information of the sample as shown in FIG. 10(b).

The grating 1004 is used in the reference arm 1020 to introduce the tilt of the order of wavelength in the reference beam, so that the required spatial carrier frequency is added to the interference signal that can allow separation of the objects term related to the sample field, its complex conjugate and the DC and auto-correlation terms when 2-D FFT of the hologram is calculated, as shown in the flow chart in FIG. 10(b). Filtering out the object term and calculating the inverse 2D-FFT yields the light field reflected by the eye at the camera detection plane.

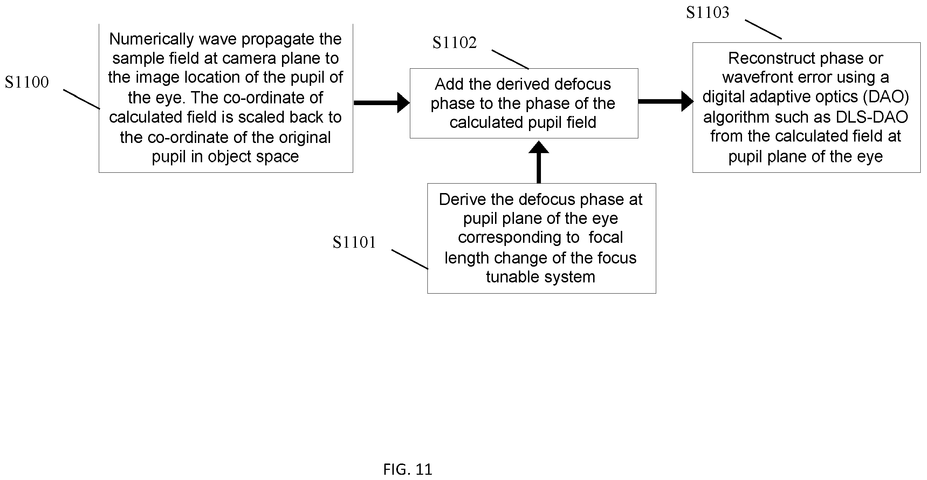

FIG. 11 shows a flowchart of the post processing steps to calculate wavefront error of the eye from the sample field obtained using the off-axis digital holography scheme according to an embodiment of the present disclosure. At S1100 the sample field is numerically wave propagated at the camera plane to the image location of the pupil of the eye. The co-ordinate of the calculated field is scaled back to the co-ordinate of the original pupil in the object space. At S1101 the defocus phase at the pupil plane of the eye is derived corresponding to the focal length change of the focus tunable system. At S1102 the derived defocus phase is added to the phase of the calculated pupil field. At S1103 the phase or wavefront error is reconstructed using a digital adaptive optics (DAO) algorithm such as DLS-DAO from the calculated field at the pupil plane of the eye.

That is, Figure 11 shows a flowchart that shows steps for the calculation of wavefront error from the extracted sample field at the camera detection plane. The sample field at the camera detection field is numerically wave propagated to the location where the image of the pupil of the eye is formed by the focus tunable optics on the camera side. The coordinate of the pupil image field is scaled back to the original coordinate of the eye's pupil plane and defocus phase corresponding to the change in the focal length of the focus tunable lens system is added to the phase of the calculated pupil field.

Finally, the resulting pupil field is processed using a digital adaptive optics technique such as DLS-DAO in order to calculate the wavefront error of the eye at the pupil plane with a high resolution comparable to the size of the camera pixel. Note that the reference arm path length can be adjusted by moving the translation stage and subsequently the coherence gate can be set at a given retinal layer, for example nerve fiber layer (NFL) or the photoreceptor, such that signals from only that particular layer where the coherence gate is set interferes with the reference light. This results in a depth resolved interference signal, which upon further post processing as shown in FIG. 10(b) and FIG. 11 can yield depth resolved wavefront error. The calculated wavefront error is in the form that is suitable for clinical applications such as wavefront guided LASIK or design of custom glasses, contact lenses and custom IOLs.

The embodiments described herein provide, inter alia, the following advantages.

Techniques and methods for determining wavefront error with respect to different planes in the eye is applied to a multimodal OCT system that can yield both anatomical and aberrometry information of the human eye. This obviates the need for separate devices for anatomical and aberrometry measurements, which can not only eliminate the inter-device errors in the measurements, but also significantly reduces the space or the device footprint, cost and time for clinicians and surgeons.

The techniques and methods offer truly personalized measurements and calculations by utilizing the real PSF field information of the human eye, which encodes the unique information about the real light field that has travelled through real anatomical refracting surfaces and optical media of the human subject's real eye.

The techniques and methods utilize a focus tunable lens system during the volumetric PSF generation using the multimodal OCT system in order to increase the SNR and the dynamic range of aberration measurements, which can significantly improve the robustness of measurements in different eyes with different anatomical and optical properties.

The methods and techniques offer personalized simulation of post-operative refraction that can be performed in real time, which can be a powerful tool in the objective evaluation of power and design of the IOL to be used for implantation in the patient's eye. This can guide the surgeons to choose the IOL with the power and design that can ensure best quality vision to the patient after surgery.

Using the numerical wave propagation technique in combination with the computational/digital adaptive optics technique such as DLS-DAO, wavefront error can be calculated at different planes without requiring any extra or additional hardware, thus saving cost and reducing system layout and design complexity.

With the adaptation of the multimodal system, such that it can be attached to or mounted on a surgical microscope and to provide intra-operative measurements, real time wavefront error calculations can be made that can guide the surgeons during surgery and help them select the correct IOL for the patient and also, particularly for toric IOL, help position and align it accurately by monitoring the quality of the PSF in real time.

Customized computer generated eye model for IOL power calculation have been demonstrated [22] [23] [24] [25] [26] [27]. However, they all use measurements from multiple separate biometric devices in order to generate a computerized geometrical eye model and use virtual ray tracing through the computer generated eye model in order to simulate the PSF. In comparison, the present disclosure, as pointed out above, uses a single multi-modal system and measures the real PSF field of the individual patient's eye, which contains the real optical and biometric properties specific to the patient's eye.

Multi-modal system that can deliver OCT imaging together with biometry or aberrometry have been demonstrated and reported for both pre-operative and intra-operative applications [12] [7] [28] [29] [30] [31]. However, they all combine devices that work on different physical principles and are often bulky, complex in design and significantly increase manufacturing costs.

The present discloure for calculation of wavefront error at different planes and IOL power utilizes a system that performs both imaging and aberrometry based on the same OCT principle, thereby reducing design complexity and cost. Also, the use of numerical wave propagation to calculate light distribution at a given plane is expected to be computationally faster than ray tracing methods.

Use of numerical wave propagation techniques to calculate light field at a given plane is well established in Fourier optics and digital holography [32] [33]. The feasibility of numerical wavefront propagation based on Fresnel approximation and PSF simulation has been demonstrated in a numerical eye model. Also, in combination with an iterative phase unwrapping technique derived from synthetic aperture radar (SAR), the wavefront error calculation and the study of the optical quality performance of some IOL designs have been shown [34] [35]. However, the measurement of input light beam is required and the reconstructed numerical eye model is based on a combination of biometric and statistical data, which uses approximations and assumptions.

Another technique uses measurement of wavefront error at one plane and scales that wavefront error at the second plane using a method based on geometrical optics approximation [36]. However, this approximation does not consider the full characteristics of the light propagation as it neglects diffraction effect and has limited accuracy when the actual wavefront is more complex and irregular [37].

Although there are several ways to perform numerical wave propagation, the present disclosure focuses on its utilization as a tool to calculate optical field in a multimodal OCT system that can generate volumetric PSF field that captures the real optical and biometric properties of each individual patients eye without any approximation or assumptions.

Also, in combination with a DAO technique the present disclosure calculates wavefront error at a given plane with high precision and high computational speed that is suitable for real time and intra-operative applications.

PSF imaging of the eye and the estimation of visual quality has been demonstrated [38] [39] [40]. However, the images provide only intensity based information and the retrieval of phase requires complex iterative numerical or digital methods [41] [42]. The present disclosure, on the other hand, directly uses the phase information of the generated PSF field and calculates wavefront error at a given plane using combination of numerical wave propagation and digital adaptive optics such DLS-DAO, which enables faster non-iterative calculations and makes it especially suitable for real-time applications.

Focus tunable lenses have been demonstrated to improve dynamic range of PSF imaging in double pass configuration, autorefractors and also improving depth of focusing in OCT imaging [43] [44] [45] [46]. However, a focus tunable system has not been implemented to increase SNR and dynamic range in a multimodal OCT system that can generate volumetric PSF scan of the eye with both amplitude and phase information as disclosed herein.

Also, according to the present disclsoure, tunable focus is used only to roughly correct primarily sphere or defocus error and optionally cylinder or astigmatism error, and these rough lower order errors are taken into account for the calculation of the total and more precise wavefront error at different planes with respect to the eye using the combination of numerical wave propagation and DAO.

Intra-operative systems for guiding the selection of IOL power and positioning it, especially axis alignment in case of toric IOL, have also been reported and demonstrated [8] [47] [48] [49] [50] [51] [52]. However, they use intra-operative measurements in a regression formula in order to calculate the IOL power and hence do not provide personalized calculation according to each individual patient's eye's real optical and biometric properties as is done in the the present disclosure.

Also, the prior art related to intra-operative toric IOL alignment rely only on wavefront measurements in a feedback loop to achieve the best possible alignment. Callisto eye system from Zeiss [53] provides digital marker during surgery but uses pre-operative keratometry and iris images for guiding the alignment. The present disclosure provides direct PSF imaging and PSF width monitoring in real time during the surgery for the toric IOL alignment, which is a more direct and reliable approach as the goal of wavefront error based vision correction procedure is to ultimately achieve the optimal PSF.

Digital holographic techniques have been applied extensively in the field of metrology and microscopy [54]. Digital holography in off-axis configuration has been proposed as wavefront sensor for retinal imaging [55]. However, the present disclosure uses a broad band light source that can provide required coherence gating for depth resolved wavefront sensing, digital adaptive optics technique such as DLS-DAO in order to extract wavefront error at the pupil plane that is suitable for any clinical application, and employs focus tunable systems for dynamic range enhancement.

More recent prior arts have demonstrated depth resolved retinal imaging using digital holography in off-axis configuration using broad band light source [56] [57] [58] [59] [60]. They have also demonstrated digital adaptive optics techniques to improve and achieve cellular level retinal imaging [61] [59]. However, the sample arm is based on a double path configuration, wherein the illumination beam entering the eye and the back reflected detection beam are of same diameter size. Due to this configuration the detected wavefront error have amplified even aberration terms and suppressed odd aberration terms, which no longer accurately represent the true wavefront error of the optics of the eye, and hence cannot be used directly for any clinical application such as wavefront guided LASIK. The present disclosure overcomes this problem by using a separate narrow beam of diameter size of around 0.6 mm illumination that avoids aberration in the incoming path and detects the wavefront error of the eye from the reflected beam passing through the full aperture of the pupil (5-7mm diameter size).

Also, the present disclosure employs focus tunable systems in combination with post-processing steps involving numerical wave propagation and digital adaptive optics technique such as DLS-DAO in order to detect accurately the wavefront error with high dynamic range at the pupil plane of the eye in the form that can be directly used for clinical applications such as wavefront guided LASIK or design of custom glasses, contact lenses and custom IOLs.

The present disclosure of the digital aberrometer based on off-axis digital holography has, inter alia, the following advantages.

The wavefront error can be obtained from the measurement of a single 2D camera shot, which enables high measurement speed in the order of hundreds of frames per second.

In comparison to point scanning systems, high speed parallel detection is enabled by a 2-D camera that provides better phase stability with respect to eye motion, which can lead to better accuracy in the calculation of wavefront error.

A very high resolution wavefront error map with resolution comparable to camera pixel size can be obtained.

The use of focus tunable system in combination with post-processing steps involving numerical wave propagation and digital adaptive optics technique such as DLS-DAO can enable wavefront error calculation with high accuracy and dynamic range.

Coherence gating enabled by the use of broad-band light source allows signal detection only from a selected depth layer in retina, thus providing depth resolved wavefront error measurement.

Moreover, the use of a narrow illumination beam enables a single path measurement, which provides the accurate representation of the wavefront error due to the optics of the eye.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations, but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any of the above-described exemplary embodiments.

It is also understood that any reference to an element herein using a designation such as "first," "second," and so forth does not generally limit the quantity or order of those elements.

Rather, these designations can be used herein as a convenient means of distinguishing between two or more elements or instances of an element. Thus, a reference to first and second elements does not mean that only two elements can be employed, or that the first element must precede the second element in some manner.

Additionally, a person having ordinary skill in the art would understand that information and signals can be represented using any of a variety of different technologies and techniques. For example, data, instructions, commands, information, signals, bits and symbols, for example, which may be referenced in the above description can be represented by voltages, currents, electromagnetic waves, magnetic fields or particles, optical fields or particles, or any combination thereof.

A skilled person would further appreciate that any of the various illustrative logical blocks, units, processors, means, circuits, methods and functions described in connection with the aspects disclosed herein can be implemented by electronic hardware (e.g., a digital implementation, an analog implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components, blocks, units, circuits, and steps have been described above generally in terms of their functionality. Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

Furthermore, a skilled person would understand that various illustrative logical blocks, units, devices, components and circuits described herein can be implemented within or performed by an integrated circuit (IC) that can include a general purpose processor, a digital signal processor (DSP), an application specific integrated circuit (ASIC), a field programmable gate array (FPGA) or other programmable logic device, or any combination thereof. The logical blocks, units, and circuits can further include antennas and/or transceivers to communicate with various components within the network or within the device. A general purpose processor can be a microprocessor, but in the alternative, the processor can be any conventional processor, controller, or state machine. A processor can also be implemented as a combination of computing devices, e.g., a combination of a DSP and a microprocessor, a plurality of microprocessors, one or more microprocessors in conjunction with a DSP core, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

In this document, the term "unit" as used herein, refers to software, firmware, hardware, and any combination of these elements for performing the associated functions described herein. Additionally, for purpose of discussion, the various units are described as discrete units; however, as would be apparent to one of ordinary skill in the art, two or more units may be combined to form a single unit that performs the associated functions according embodiments of the present disclosure.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure. For example, functionality illustrated to be performed by separate processing logic elements, or controllers, may be performed by the same processing logic element, or controller. Hence, references to specific functional units are only references to a suitable means for providing the described functionality, rather than indicative of a strict logical or physical structure or organization.

Various modifications to the implementations described in this disclosure will be readily apparent to those skilled in the art, and the general principles defined herein can be applied to other implementations without departing from the scope of this disclosure. Thus, the disclosure is not intended to be limited to the implementations shown herein, but is to be accorded the widest scope consistent with the novel features and principles disclosed herein, as recited in the claims below.

### List of references

1. N. Maeda, "Clinical applications of wavefront aberrometry - a review," Clin. Experiment. Ophthalmol. 37, 118-129 (2009).
2. G. Dai, Wavefront Optics for Vision Correction (SPIE Press, 2008).
3. J. J. Rozema, D. E. M. Van Dyck, and M.-J. Tassignon, "Clinical comparison of 6 aberrometers. Part 1: Technical specifications," J. Cataract Refract. Surg. 31, 1114-1127 (2005).
4. J. J. Rozema, D. E. M. Van Dyck, and M.-J. Tassignon, "Clinical comparison of 6 aberrometers Part 2: Statistical comparison in a test group," J. Cataract Refract. Surg. 32, 33-44 (2006).
5. M. G. Woodcock, W. F. Wiley, K. Assil, and S. S. Lane, "Uses for Intraoperative Wavefront Aberrometry," 7 (n.d.).
6. A. Y. V. Heugten and D. S. Durrie, "Integrated surgical microscope and wavefront sensor," United States patent US9107612B2 (August 18, 2015).
7. Y. Zhou, B. Chew, and W. Shea, "Apparatus and method for operating a real time large dipoter range sequential wavefront sensor," United States patent US9101292B2 (August 11, 2015).
8. T. lanchulev, "Intraoperative estimation of intraocular lens power," United States patent US7556378B1 (July 7, 2009).
9. O. Findl, M. Trost, N. Hirnschall, M. Volkwardt, F. Bajramovic, and T. Teuber, "Method for selecting an iol on the basis of the prediction of the anatomical, post-operative position and orientation thereof," United States patent US20180368970A1 (December 27, 2018).
10. C. Hauger, H. Matz, M. Seesselberg, A. Seiwert, M. Wilzbach, X. Wei, O. Findl, and N. Hirnschall, "Method, ophthalmic measuring system and computer-readable storage medium for selecting an intraocular lens," European Union patent EP2796087A1 (October 29, 2014).
11. N. Hirnschall, S. Amir-Asgari, S. Maedel, and O. Findl, "Predicting the Postoperative Intraocular Lens Position Using Continuous Intraoperative Optical Coherence Tomography Measurements," Invest. Ophthalmol. Vis. Sci. 54, 5196-5203 (2013).
12. A. N. Artsyukhovich, Z. A. Aslan, L. Yu, and M. Boukhny, "Integrated oct-refractometer system for ocular biometry," United States patent US20170079522A1 (March 23, 2017).
13. S. Ortiz, P. Pérez-Merino, S. Durán, M. Velasco-Ocana, J. Birkenfeld, A. de Castro, I. Jiménez-Alfaro, and S. Marcos, "Full OCT anterior segment biometry: an application in cataract surgery," Biomed. Opt. Express 4, 387-396 (2013).
14. "Intraocular Lens Power Calculation - Still Searching for the Holy Grail," touchOPHTHALMOLOGY (2015).
15. S. Ortiz, D. Siedlecki, P. Pérez-Merino, N. Chia, A. de Castro, M. Szkulmowski, M. Wojtkowski, and S. Marcos, "Corneal topography from spectral optical coherence tomography (sOCT)," Biomed. Opt. Express 2, 3232-3247 (2011).
16. K. Karnowski, B. J. Kaluzny, M. Szkulmowski, M. Gora, and M. Wojtkowski, "Corneal topography with high-speed swept source OCT in clinical examination," Biomed. Opt. Express 2, 2709-2720 (2011).
17. Y.-Z. Liu, F. A. South, Y. Xu, P. S. Carney, and S. A. Boppart, "Computational optical coherence tomography [Invited]," Biomed. Opt. Express 8, 1549-1574 (2017).
18. A. Kumar, L. M. Wurster, M. Salas, L. Ginner, W. Drexler, and R. A. Leitgeb, "In-vivo digital wavefront sensing using swept source OCT," Biomed. Opt. Express 8, 3369-3382 (2017).
19. F. A. South, Y.-Z. Liu, A. J. Bower, Y. Xu, P. S. Carney, and S. A. Boppart, "Wavefront measurement using computational adaptive optics," JOSA A 35, 466-473 (2018).
20. A. Kumar, "Optical Apparatus," U.S. patent WO/2019/010507 (January 17, 2019).
21. R. Bergner, R. Barth, A. Doering, F. Behrendt, and K.-D. Voigt, "Method for determining distances in the anterior ocular segment," Canada patent CA2417776C (June 12, 2012).
22. P. Rosales and S. Marcos, "Customized computer models of eyes with intraocular lenses," Opt. Express 15, 2204-2218 (2007).
23. C. Canovas and P. Artal, "Customized eye models for determining optimized intraocular lenses power," Biomed. Opt. Express 2, 1649-1662 (2011).
24. C. C. Vidal, P. Artal, M. V. D. Mooren, and P. A. Piers, "Customized intraocular lens power calculation system and method," United States patent US8746882B2 (June 10, 2014).
25. S. Salvati, C. Tanassi, G. Meneghini, R. FRISON, and W. ZANETTE, "Systems and methods for implanting and examining intraocular lens," United States patent US8216305B2 (July 10, 2012).
26. T. Olsen, "System and method for determining and predicting iol power in situ," World Intellectual Property Organization patent WO2010028654A1 (March 18, 2010).
27. T. Bühren, M. Trost, C. Weth, F. Bajramovic, W.-J. Chen, M. Volkwardt, and M. Zimmermann, "Method for optimized selection of the IOL to be implanted in an eye," Germany patent DE102013020706A1 (June 11, 2015).
28. D. Neal, T. D. Raymond, R. J. Copland, W. Xiong, P. D. Pulaski, S. Farrer, C. C. Vidal, and D. Hamrick, "Optical imaging and measurement systems and methods for cataract surgery and treatment planning," United States patent US10506923B2 (December 17, 2019).
29. Y. Zhou and W. Shea, "Large diopter range real time sequential wavefront sensor," United States patent US8356900B2 (January 22, 2013).
30. B. Ke, X. Mao, H. Jiang, J. He, C. Liu, M. Li, Y. Yuan, and J. Wang, "The Relationship Between High-Order Aberration and Anterior Ocular Biometry During Accommodation in Young Healthy Adults," Invest. Ophthalmol. Vis. Sci. 58, 5628-5635 (2017).
31. G. Belloni, B. Chou, L. Meza, Y.-C. Chang, H. A. Durkee, J.-M. A. Parel, F. Manns, and M. Ruggeri, "Evaluation of a combined anterior segment OCT and aberrometer using the same light source," Invest. Ophthalmol. Vis. Sci. 61, 1705-1705 (2020).
32. J. W. Goodman, Introduction to Fourier Optics (Roberts and Company Publishers, 2005).
33. T.-C. Poon and J.-P. Liu, Introduction to Modern Digital Holography: With Matlab (Cambridge University Press, 2014).
34. J. Pérez, D. Mas, C. Illueca *, J. J. Miret, C. Vázquez, and C. Hernández, "Complete algorithm for the calculation light patterns inside the ocular media," J. Mod. Opt. 52, 1161-1176 (2005).
35. J. Rouarch, J. Espinosa, J. J. Miret, D. Mas, J. Pérez, and C. Illueca, "Propagation and phase reconstruction of ocular wavefronts with SAR techniques," J. Mod. Opt. 55, 717-725 (2008).
36. G. Dai, "Wavefront propagation from one plane to another," World Intellectual Property Organization patent WO2008039604A3 (April 16, 2009).
37. G. Dai, C. E. Campbell, L. Chen, H. Zhao, and D. Chernyak, "Wavefront propagation from one plane to another with the use of Zernike polynomials and Taylor monomials," Appl. Opt. 48, 477-488 (2009).
38. R. Navarro and M. A. Losada, "Phase transfer and point-spread function of the human eye determined by a new asymmetric double-pass method," J. Opt. Soc. Am. A 12, 2385 (1995).
39. P. Artal, S. Marcos, D. R. Williams, and R. Navarro, "Odd aberrations and double-pass measurements of retinal image quality," J. Opt. Soc. Am. A 12, 195 (1995).
40. J. Liang and G. Westheimer, "Optical performances of human eyes derived from double-pass measurements," JOSA A 12, 1411-1416 (1995).
41. I. Iglesias, N. López-Gil, and P. Artal, "Reconstruction of the point-spread function of the human eye from two double-pass retinal images by phase-retrieval algorithms," JOSA A 15, 326-339 (1998).
42. J. Santamaría, P. Artal, and J. Bescós, "Determination of the point-spread function of human eyes using a hybrid optical-digital method," J. Opt. Soc. Am. A 4, 1109-14 (1987).
43. F. Sanàbria, F. Díaz-Doutón, M. Aldaba, and J. Pujol, "Spherical refractive correction with an electro-optical liquid lens in a double-pass system," J. Eur. Opt. Soc. - Rapid Publ. 8, (2013).
44. S. R. DAVE, D. Lim, and N. J. DURR, "Tunable-lens-based refractive examination," World Intellectual Property Organization patent WO2017218539A1 (December 21, 2017).
45. G. Li, Z. Han, and G. Lan, "Adaptive Electro-Optic Lenses for Vision Correction and Assessment, and Eye Imaging," Invest. Ophthalmol. Vis. Sci. 53, 3584-3584 (2012).
46. J. P. Ehlers, Y. K. Tao, and S. K. Srivastava, "Microscope-integrated OCT system with an electrically tunable focus," United States patent US9848770B2 (December 26, 2017).
47. T. lanchulev, J. Salz, K. Hoffer, T. Albini, H. Hsu, and L. Labree, "Intraoperative optical refractive biometry for intraocular lens power estimation without axial length and keratometry measurements," J. Cataract Refract. Surg. 31, 1530-1536 (2005).
48. K. M. Hatch, E. C. Woodcock, and J. H. Talamo, "Intraocular Lens Power Selection and Positioning With and Without Intraoperative Aberrometry," J. Refract. Surg. 31, 237-242 (2015).
49. T. P. Page, "Intraoperative aberrometry-assisted toric IOL exchange following unexpected surgically induced astigmatism," JCRS Online Case Rep. 2, e25-e29 (2014).
50. Y. Zhou, W. Shea, B. Linder, and B. Phil, "Optimizing vision correction procedures," United States patent US8454162B2 (June 4, 2013).
51. T. D. Padrick, J. T. Holladay, D. B. Tran, A. K. Plumley, R. J. Michaels, and J. Padgett, "Optical angular measurement system for ophthalmic applications and method for positioning of a toric intraocular lens with increased accuracy," World Intellectual Property Organization patent WO2010054268A2 (May 14, 2010).
52. A. S. Hura and R. H. Osher, "Comparing the Zeiss Callisto Eye and the Alcon Verion Image Guided System Toric Lens Alignment Technologies," J. Refract. Surg. Thorofare NJ 1995 33, 482-487 (2017).
53. "ZEISS CALLISTO Eye - Computer assisted cataract surgery - ZEISS Medical Technology | ZEISS International," https://www.zeiss.com/meditec/int/product-portfolio/surgical-microscopes/ophthalmic-microscopes/callisto-eye-markerless-toric-iol-alignment.html.
54. U. Schnars, C. Falldorf, J. Watson, and W. Jüptner, Digital Holography and Wavefront Sensing: Principles, Techniques and Applications (Springer, 2014).
55. C. Liu and M. K. Kim, "Digital holographic adaptive optics for ocular imaging: proof of principle," Opt. Lett. 36, 2710-2712 (2011).
56. H. Sudkamp, P. Koch, H. Spahr, D. Hillmann, G. Franke, M. Münst, F. Reinholz, R. Birngruber, and G. Hüttmann, "In-vivo retinal imaging with off-axis full-field time-domain optical coherence tomography," Opt. Lett. 41, 4987-4990 (2016).
57. H. Spahr, H. SUDKAMP, G. Hüttmann, P. Koch, G. Franke, D. Hillmann, and R. Birngruber, "Verfahren und Vorrichtung zum Ablichten wenigstens einer Schnittfläche im Innern eines Licht streuenden Objekts," Germany patent DE102015113465A1 (February 16, 2017).
58. D. Hillmann, H. Spahr, H. Sudkamp, C. Hain, L. Hinkel, G. Franke, and G. Hüttmann, "Off-axis reference beam for full-field swept-source OCT and holoscopy," Opt. Express 25, 27770 (2017).
59. L. Ginner, T. Schmoll, A. Kumar, M. Salas, N. Pricoupenko, L. M. Wurster, and R. A. Leitgeb, "Holographic line field en-face OCT with digital adaptive optics in the retina in vivo," Biomed. Opt. Express 9, 472-485 (2018).
60. P. Koch, G. Franke, H. Spahr, H. Sudkamp, G. Hüttmann, D. Hillmann, and R. Birngruber, "Method and device for exposing at least one sectional face in the interior of a light-scattering object," World Intellectual Property Organization patent WO2017029160A1 (February 23, 2017).
61. H. Sudkamp, D. Hillmann, P. Koch, M. vom Endt, H. Spahr, M. Münst, C. Pfäffle, R. Birngruber, and G. Hüttmann, "Simple approach for aberration-corrected OCT imaging of the human retina," Opt. Lett. 43, 4224-4227 (2018).

## Claims

1. Optical apparatus, comprising:
a source of wavelength tunable laser light (100) or a broad band partially coherent light source (1001),
wherein light from the wavelength tunable laser light (100) or the broad band partially coherent light source (1001) is split into a sample arm (110, 1010) as illumination light and a reference arm (120, 1020) as reference light,
the sample arm (110, 1010) comprising:
a first beam splitter (111, 1011),
means for directing the illumination light via the first beam splitter (111, 1011) as a light spot to a sample, wherein an image of the light spot is reflected from the sample,
a focus tunable optics (112, 1012) receiving the image of the light spot from the sample after being transmitted through the first beam splitter (111, 1011) and focusing the image to a detection plane,
wherein a photodetector unit is adapted for receiving the recombined light from the sample arm (110, 1010) and the reference arm (120, 1020),
wherein the sample arm (110, 1010) comprises a separate illumination channel configured to inject light via a mirror (116, 1003) and the first beam splitter (111, 1011) to the sample without passing through the focus tunable optics (112, 1012).

2. Optical apparatus according to claim 1, wherein the focus tunable optics (112, 1012) is configured to be controlled manually, wherein the focus tunable optics (112, 1012) preferably comprise a plurality of lenses arranged in a Badal system, such that the image is focused to the detection plane and/or wherein the focus tunable optics (112, 1012) is configured to be controlled automatically, wherein the focus tunable optics (112, 1012) preferably comprise electrically focus tunable liquid crystal optical elements, such that the image is focused to the detection plane, wherein preferably the focus tunable optics (112, 1012) is adapted to increase the dynamic range of the signal detection more preferably adapted to compensate defocus and/or astigmatism of the sample.

3. Optical apparatus according to claim 1or 2, wherein the sample is an eye (200) and wherein the focus tunable optics (112) is adapted to ensure that the retinal plane (202) of the eye (200) is continuously conjugated to or imaged at the detection plane.

4. Optical apparatus according to any one of claims 1 to 3, further comprising a computing unit (140) being connected to the photodetector unit, wherein the computing unit (140) is configured for digitizationand further data processing.

5. Optical apparatus according to any one of claims 1 to 4, wherein the sample arm (110) further comprises a scanner, preferably a 2-D scanner (113) placed at the Fourier plane of collimation optics (114) located in front of the detection plane.

6. Optical apparatus according to any one of claims 1 to 5, wherein the sample arm (110) further comprises a detection fiber (117) being adapted to receive the image of the illuminated spot at the detection plane and guide light to the photodetector and more preferably an actuator (170) configured to translate the detection fiber (117) laterally across the image of the illuminated spot.

7. Optical apparatus according to any one of claims 4 to 6, wherein the computing unit (140, 1040) is configured to generate a volume image of the sample preferably a full eye (200, 1200) and point spread functions, PSF and to determine a wavefront error using a digital adaptive optics, DAO algorithm preferably a digital lateral shearing based digital adaptive optics algorithm, DLS-DAO algorithm.

8. Optical apparatus according to claim 7, wherein the computing unit (140) is configured to:
obtain volumetric optical coherence tomography data, OCT data of PSF scans of the eye (200),
extract an enface PSF field at a retinal layer of the eye (200) after OCT based data processing,
derive a defocus distance in an image space from a shift in the image plane at which an image of the light spot is best focused when the focal length of the focus tunable optics (112) is changed,
calculate 2-D FFT of the PSF field and add a defocus phase corresponding to the derived defocused distance to the phase of the calculated Fourier field,
numerically wave propagate the resulting field at Fourier plane to the image location of the pupil (201) of the eye (200), and
reconstruct the phase or wavefront error using the digital adaptive optics, DAO algorithm, preferably the DSL-DAO algorithm from the calculated field at a pupil plane (201) of the eye (200).

9. Optical apparatus according to claim 7, wherein the computing unit (140) is configured to:
obtain volumetric optical coherence tomography data, OCT data of PSF scans of the eye (200),
extract an enface PSF field at a retinal layer of the eye after OCT based data processing,
calculate 2-D FFT of the PSF field,
numerically wave propagate the resulting field at Fourier plane to the image location of the pupil (201) of the eye (200),
derive a defocus distance of a focal plane of the eye (200) from the retina, from a change in focus of the focus tunable optics (112),
add a defocus phase corresponding to the derived defocused distance to the phase of the calculated pupil field, and
reconstruct the phase or wavefront error using the digital adaptive optics, DAO algorithm, preferably the DSL-DAO algorithm from the calculated field at a pupil plane (201) of the eye (200).

10. Optical apparatus according to claim 7, wherein the computing unit (140) is configured to:
obtain volumetric optical coherence tomography data, OCT data of PSF scans of the eye (200) in an aphakic state,
extract an enface PSF field at a retinal layer after OCT based data processing,
derive a defocus distance of a focal plane of the aphakic eye (200) from the retina, from a change in focus of the focus tunable optics (112),
numerically wave propagate the PSF field to an estimated intraocular lens, IOL, location plane (209) within the eye (200) taking into account the defocus distance, and
reconstruct the phase or wavefront error using the digital adaptive optics, DAO algorithm, preferably the DSL-DAO algorithm from the calculated field at the IOL location plane(209) within the eye (200).

11. Optical apparatus according to claim 10, wherein the computing unit (140) is configured to determine at least one of a sphere, a cylinder and cylinder axis values from the reconstructed phase error and to use the determined values for selecting an IOL (180) or for designing a custom IOL (180) having an optimal sphere and cylinder power and cylinder axis for correcting the phase or wavefront error of the eye (200).

12. Optical apparatus according to claim 10 or 11, wherein the computing unit (140) is configured to evaluate an IOL (180) performance preferably comprising:
multiplying a field at the IOL location plane (209) with a complex exponential of a known phase of a selected IOL (180),
calculating the field back at the retinal plane (202) using a numerical wave propagation algorithm, and
calculating a spot size and a modulus transfer function, MTF for quantifying a visual performance.

13. Optical apparatus according to claim 7, wherein the computing unit (140) is configured to:
obtain volumetric optical coherence tomography data, OCT data of PSF scans of the eye (200) in a phakic state,
extract an enface PSF field at a retinal layer after OCT based data processing,
derive a defocus distance of a focal plane of the phakic eye (200) from the retina, from a change in focus of the focus tunable optics (112),
numerically wave propagate the PSF field to a plane at a last surface of a crystalline lens considering vitreous media within the eye (200) and taking account a defocus distance,
multiply the calculated field with a complex conjugate of a transmission function of the crystalline lens to cancel its refractive effect,
numerically wave propagate the resulting field to an estimated intraocular lens, IOL, location (209), and
reconstruct the phase or wavefront error using the digital adaptive optics, DAO algorithm, preferably the DSL-DAO algorithm from the calculated field at an IOL location plane (209) within the eye.

14. Optical apparatus according to claim 13, wherein the computing unit (140) is configured to determine at least one of a sphere, a cylinder and cylinder axis values from the reconstructed phase error and to use the determined values for selecting an IOL (180) or for designing a custom IOL (180) having an optimal sphere and cylinder power and cylinder axis for correcting the phase or wavefront error of the eye (200).

15. Optical apparatus according to claim 13, wherein the computing unit (140) is configured to evaluate an IOL performance preferably comprising:
multiplying a field at the IOL location plane (209) with the transmission function of a selected IOL (180),
calculating the field back at the retinal plane (202) using a numerical wave propagation algorithm and
calculating a spot size and a modulus transfer function, MTF for quantifying a visual performance.

16. Optical apparatus according to any one of claims 1 to 15, wherein the computing unit (140, 1040) is configured to:
provide PSF corresponding to the retinal layer and wavefront error of an eye (200, 1200),
determine cylinder power and axis of an astigmatism or cylinder error correcting toric intraocular lens, IOL,
determine residual cylinder error after toric IOL is implanted in an eye (200, 1200) during the IOL implant surgery from a quality metric based on spot size of the PSF profile,
determine angle by which toric IOL needs to be rotated to achieve optimal axis alignment in order to cancel the residual cylinder error, and
finally confirm the accuracy of the axis alignment of the toric IOL based on a quality metric based on spot size of the PSF profile.

## Patentansprüche

1. Optische Vorrichtung, aufweisend:
eine Quelle für wellenlängeneinstellbares Laserlicht (100) oder eine teilkohärente Breitbandlichtquelle (1001),
wobei Licht von dem wellenlängeneinstellbaren Laserlicht (100) oder der teilkohärenten Breitbandlichtquelle (1001) in einen Probenarm (110, 1010) als Beleuchtungslicht und einen Referenzarm (120, 1020) als Referenzlicht aufgeteilt wird,
wobei der Probenarm (110, 1010), aufweist:
einen ersten Strahlteiler (111, 1011),
Mittel zum Lenken des Beleuchtungslichts über den ersten Strahlteiler (111, 1011) als einen Lichtpunkt zu einer Probe, wobei ein Bild des Lichtpunkts von der Probe reflektiert wird,
eine fokuseinstellbare Optik (112, 1012), die das Bild des Lichtpunkts von der Probe nach der Übertragung durch den ersten Strahlteiler (111, 1011) empfängt und das Bild auf eine Detektionsebene fokussiert,
wobei eine Photodetektoreinheit zum Empfangen des rekombinierten Lichts von dem Probenarm (110, 1010) und dem Referenzarm (120, 1020) angepasst ist,
wobei der Probenarm (110, 1010) einen separaten Beleuchtungskanal aufweist, der konfiguriert ist, um Licht über einen Spiegel (116, 1003) und den ersten Strahlteiler (111, 1011) zu der Probe zu einzuspeisen, ohne die fokuseinstellbare Optik (112, 1012) zu durchlaufen.

2. Optische Vorrichtung nach Anspruch 1, wobei die fokuseinstellbare Optik (112, 1012) konfiguriert ist, um manuell gesteuert zu werden, wobei die fokuseinstellbare Optik (112, 1012) vorzugsweise mehrere Linsen aufweist, die in einem Badal-System derart angeordnet sind, dass das Bild auf die Detektionsebene fokussiert wird und/oder wobei die fokuseinstellbare Optik (112, 1012) konfiguriert ist, um automatisch gesteuert zu werden, wobei die fokuseinstellbare Optik (112, 1012) vorzugsweise elektrisch fokuseinstellbare Flüssigkristall-Optikelemente derart aufweist, dass das Bild auf die Detektionsebene fokussiert wird, wobei vorzugsweise die fokuseinstellbare Optik (112, 1012) angepasst ist, um den Dynamikbereich der Signaldetektion zu erhöhen, mehr bevorzugt angepasst ist, um eine Defokussierung und/oder einen Astigmatismus der Probe zu kompensieren.

3. Optische Vorrichtung nach Anspruch 1 oder 2, wobei die Probe ein Auge (200) ist und wobei die fokuseinstellbare Optik (112) angepasst ist, um sicherzustellen, dass die Netzhautebene (202) des Auges (200) kontinuierlich zu der Detektionsebene konjugiert oder auf diese abgebildet wird.

4. Optische Vorrichtung nach einem der Ansprüche 1 bis 3, ferner aufweisend eine Rechnereinheit (140), die mit der Photodetektoreinheit verbunden ist, wobei die Rechnereinheit (140) für eine Digitalisierung und weitere Datenverarbeitung konfiguriert ist.

5. Optische Vorrichtung nach einem der Ansprüche 1 bis 4, wobei der Probenarm (110) ferner einen Scanner aufweist, vorzugsweise einen 2-D-Scanner (113), der in der Fourier-Ebene einer Kollimationsoptik (114) platziert ist, die sich vor der Detektionsebene befindet.

6. Optische Vorrichtung nach einem der Ansprüche 1 bis 5, wobei der Probenarm (110) ferner eine Detektionsfaser (117) aufweist, die angepasst ist, um das Bild des beleuchteten Punkts in der Detektionsebene zu empfangen und Licht zu dem Photodetektor zu leiten, und mehr bevorzugt einen Aktuator (170), der konfiguriert ist, um die Detektionsfaser (117) lateral über das Bild des beleuchteten Punkts zu bewegen.

7. Optische Vorrichtung nach einem der Ansprüche 4 bis 6, wobei die Rechnereinheit (140, 1040) konfiguriert ist, um ein Volumenbild der Probe, vorzugsweise eines vollständigen Auges (200, 1200), und Punktspreizfunktionen, PSF, zu erzeugen und einen Wellenfrontfehler unter Verwendung eines digitalen adaptive Optik-Algorithmus, DAO-Algorithmus, vorzugsweise eines auf digitaler lateraler Scherung basierenden digitalen adaptive Optik-Algorithmus, DLS-DAO-Algorithmus, zu bestimmen.

8. Optische Vorrichtung nach Anspruch 7, wobei die Rechnereinheit (140) konfiguriert ist zum:
Erhalten volumetrischer optischer Kohärenztomographie-Daten, OCT-Daten, von PSF-Scans des Auges (200),
Extrahieren eines En-face-PSF-Feldes an einer Netzhautschicht des Auges (200) nach OCT-basierter Datenverarbeitung,
Ableiten einer Defokussierungsdistanz in einem Bildraum aus einer Verschiebung in der Bildebene, bei der ein Bild des Lichtpunkts am besten fokussiert ist, wenn die Brennweite der fokuseinstellbaren Optik (112) verändert wird,
Berechnen der 2-D-FFT des PSF-Feldes und Addieren einer Defokussierungsphase entsprechend der abgeleiteten defokussierten Distanz zu der Phase des berechneten Fourier-Feldes,
numerisches Wellenausbreiten des resultierenden Feldes an der Fourier-Ebene zu der Bildposition der Pupille (201) des Auges (200), und
Rekonstruieren des Phasen- oder Wellenfrontfehlers unter Verwendung des digitalen adaptive Optik-Algorithmus, DAO-Algorithmus, vorzugsweise des DSL-DAO-Algorithmus aus dem berechneten Feld an einer Pupillenebene (201) des Auges (200).

9. Optische Vorrichtung nach Anspruch 7, wobei die Rechnereinheit (140) konfiguriert ist zum:
Erhalten volumetrischer optischer Kohärenztomographie-Daten, OCT-Daten, von PSF-Scans des Auges (200),
Extrahieren eines En-face-PSF-Feldes an einer Netzhautschicht des Auges nach OCT-basierter Datenverarbeitung,
Berechnen des 2-D-FFT des PSF-Feldes,
numerisches Wellenausbreiten des resultierenden Feldes an der Fourier-Ebene zu der Bildposition der Pupille (201) des Auges (200),
Ableiten einer Defokussierungsdistanz einer Brennebene des Auges (200) von der Netzhaut aus einer Änderung der Fokussierung der fokuseinstellbaren Optik (112),
Addieren einer Defokussierungsphase entsprechend der abgeleiteten defokussierten Distanz zu der Phase des berechneten Pupillenfeldes, und
Rekonstruieren des Phasen- oder Wellenfrontfehlers unter Verwendung des digitalen adaptive Optik-Algorithmus, DAO-Algorithmus, vorzugsweise des DSL-DAO-Algorithmus aus dem berechneten Feld an einer Pupillenebene (201) des Auges (200).

10. Optische Vorrichtung nach Anspruch 7, wobei die Rechnereinheit (140) konfiguriert ist zum:
Erhalten volumetrischer optischer Kohärenztomographie-Daten, OCT-Daten, von PSF-Scans des Auges (200) in einem aphaken Zustand,
Extrahieren eines En-face-PSF-Feldes an einer Netzhautschicht nach OCT-basierter Datenverarbeitung,
Ableiten einer Defokussierungsdistanz einer Brennebene des aphaken Auges (200) von der Netzhaut aus einer Änderung der Fokussierung der fokuseinstellbaren Optik (112),
numerisches Wellenausbreiten des PSF-Feldes zu einer geschätzten Intraokularlinsenlageebene (209), IOL-Lageebene, innerhalb des Auges (200) unter Berücksichtigung der Defokussierungsdistanz, und
Rekonstruieren des Phasen- oder Wellenfrontfehlers unter Verwendung des digitalen adaptive Optik-Algorithmus, DAO-Algorithmus, vorzugsweise des DSL-DAO-Algorithmus aus dem berechneten Feld an der IOL-Lageebene (209) innerhalb des Auges (200).

11. Optische Vorrichtung nach Anspruch 10, wobei die Rechnereinheit (140) konfiguriert ist, um mindestens einen von einem Kugel-, einem Zylinder- und einem Zylinderachsenwert aus dem rekonstruierten Phasenfehler zu bestimmen und die bestimmten Werte zum Auswählen einer IOL (180) oder zum Gestalten einer kundenspezifischen IOL (180) mit einer optimalen Kugel- und Zylinderstärke und Zylinderachse zum Korrigieren des Phasen- oder Wellenfrontfehlers des Auges (200) zu verwenden.

12. Optische Vorrichtung nach Anspruch 10 oder 11, wobei die Rechnereinheit (140) konfiguriert ist, um die Leistung einer IOL (180) zu bewerten, vorzugsweise aufweisend:
Multiplizieren eines Feldes an der IOL-Lageebene (209) mit einer komplexen Exponentialfunktion einer bekannten Phase einer ausgewählten IOL (180),
Zurückberechnen des Feldes an der Netzhautebene (202) unter Verwendung eines numerischen Wellenausbreitungsalgorithmus, und
Berechnen einer Punktgröße und einer Modulübertragungsfunktion, MTF, zum Quantifizieren einer visuellen Leistung.

13. Optische Vorrichtung nach Anspruch 7, wobei die Rechnereinheit (140) konfiguriert ist zum:
Erhalten volumetrischer optischer Kohärenztomographie-Daten, OCT-Daten, von PSF-Scans des Auges (200) in einem phaken Zustand,
Extrahieren eines En-face-PSF-Feldes an einer Netzhautschicht nach OCT-basierter Datenverarbeitung,
Ableiten einer Defokussierungsdistanz einer Brennebene des phaken Auges (200) von der Netzhaut aus einer Änderung der Fokussierung der fokuseinstellbaren Optik (112),
numerisches Wellenausbreiten des PSF-Feldes zu einer Ebene an einer letzten Oberfläche einer kristallinen Linse unter Berücksichtigung von Glaskörpermedien innerhalb des Auges (200) und unter Berücksichtigung einer Defokussierungsdistanz,
Multiplizieren des berechneten Feldes mit einem komplexen Konjugat einer Übertragungsfunktion der kristallinen Linse, um deren Brechungseffekt zu tilgen,
numerisches Wellenausbreiten des resultierendes Feldes zu einer geschätzten Intraokularlinsenlage (209), IOL-Lage, und
Rekonstruieren des Phasen- oder Wellenfrontfehlers unter Verwendung des digitalen adaptive Optik-Algorithmus, DAO-Algorithmus, vorzugsweise des DSL-DAO-Algorithmus aus dem berechneten Feld an einer IOL-Lageebene (209) innerhalb des Auges.

14. Optische Vorrichtung nach Anspruch 13, wobei die Rechnereinheit (140) konfiguriert ist, um mindestens einen von einem Kugel-, einem Zylinder- und einem Zylinderachsenwert aus dem rekonstruierten Phasenfehler zu bestimmen und die bestimmten Werte zum Auswählen einer IOL (180) oder zum Gestalten einer individuellen IOL (180) mit einer optimalen Kugel- und Zylinderstärke und Zylinderachse zum Korrigieren des Phasen- oder Wellenfrontfehlers des Auges (200) zu verwenden.

15. Optische Vorrichtung nach Anspruch 13, wobei die Rechnereinheit (140) konfiguriert ist, um eine IOL-Leistung zu bewerten, vorzugsweise aufweisend:
Multiplizieren eines Feldes an der IOL-Lageebene (209) mit der Übertragungsfunktion einer ausgewählten IOL (180),
Zurückberechnen des Feldes an der Netzhautebene (202) unter Verwendung eines numerischen Wellenausbreitungsalgorithmus und
Berechnen einer Punktgröße und einer Modulübertragungsfunktion, MTF, zum Quantifizieren einer visuellen Leistung.

16. Optische Vorrichtung nach einem der Ansprüche 1 bis 15, wobei die Rechnereinheit (140, 1040) konfiguriert ist zum:
Bereitstellen einer PSF, die der Netzhautschicht und dem Wellenfrontfehler eines Auges (200, 1200) entspricht,
Bestimmen der Zylinderstärke und -achse einer Astigmatismus oder Zylinderfehler korrigierenden torischen Intraokularlinse, IOL,
Bestimmen des verbleibenden Zylinderfehlers nach Implantation einer torischen IOL in ein Auge (200, 1200) während der IOL-Implantationschirurgie aus einer Qualitätsmetrik basierend auf der Punktgröße des PSF-Profils,
Bestimmen eines Winkels, um den die torische IOL gedreht werden muss, um eine optimale Achsenausrichtung zu erreichen, um den verbleibenden Zylinderfehler zu tilgen, und
Abschließendes Bestätigen der Genauigkeit der Achsenausrichtung der torischen IOL basierend auf einer Qualitätsmetrik basierend auf der Fleckgröße des PSF-Profils.

## Revendications

1. Appareil optique, comprenant :
une source de lumière laser accordable en longueur d'onde (100) ou une source de lumière partiellement cohérente à large bande (1001),
dans lequel de la lumière provenant de la lumière laser accordable en longueur d'onde (100) ou de la source de lumière partiellement cohérente à large bande (1001) est divisée en un bras d'échantillon (110, 1010) sous la forme de lumière d'illumination et en un bras de référence (120, 1020) sous la forme de lumière de référence,
le bras d'échantillon (110, 1010) comprenant :
un premier diviseur de faisceau (111, 1011),
un moyen pour diriger la lumière d'illumination par l'intermédiaire du premier diviseur de faisceau (111, 1011) sous la forme d'un point lumineux vers un échantillon, dans lequel une image du point lumineux est réfléchie par l'échantillon,
une optique à focalisation accordable (112, 1012) recevant l'image du point lumineux en provenance de l'échantillon après sa transmission à travers le premier diviseur de faisceau (111, 1011) et focalisant l'image sur un plan de détection,
dans lequel une unité de photodétecteur est adaptée pour recevoir la lumière recombinée en provenance du bras d'échantillon (110, 1010) et du bras de référence (120, 1020),
dans lequel le bras d'échantillon (110, 1010) comprend un canal d'illumination séparé configuré pour injecter de la lumière par l'intermédiaire d'un miroir (116, 1003) et du premier diviseur de faisceau (111, 1011) vers l'échantillon sans passer par l'optique à focalisation accordable (112, 1012).

2. Appareil optique selon la revendication 1, dans lequel l'optique à focalisation accordable (112, 1012) est configurée pour être commandée manuellement, dans lequel l'optique à focalisation accordable (112, 1012) comprend de préférence une pluralité de lentilles agencées dans un système de Badal, de telle sorte que l'image est focalisée sur le plan de détection et/ou dans lequel l'optique à focalisation accordable (112, 1012) est configurée pour être commandée automatiquement, dans lequel l'optique à focalisation accordable (112, 1012) comprend de préférence des éléments optiques à cristaux liquides à focalisation accordable électriquement, de telle sorte que l'image est focalisée sur le plan de détection, dans lequel l'optique à focalisation accordable (112, 1012) est de préférence adaptée pour augmenter la gamme dynamique de la détection de signal, et plus préférablement adaptée pour compenser une défocalisation et/ou un astigmatisme de l'échantillon.

3. Appareil optique selon la revendication 1 ou 2, dans lequel l'échantillon est un œil (200) et dans lequel l'optique à focalisation accordable (112) est adaptée pour garantir que le plan rétinien (202) de l'œil (200) est continuellement conjugué au plan de détection ou imagé au niveau de ce dernier.

4. Appareil optique selon l'une quelconque des revendications 1 à 3, comprenant en outre une unité de calcul (140) connectée à l'unité de photodétecteur, dans lequel l'unité de calcul (140) est configurée pour la numérisation et le traitement ultérieur de données.

5. Appareil optique selon l'une quelconque des revendications 1 à 4, dans lequel le bras d'échantillon (110) comprend en outre un dispositif de balayage, de préférence un dispositif de balayage 2D (113) placé au niveau du plan de Fourier de l'optique de collimation (114) située devant le plan de détection.

6. Appareil optique selon l'une quelconque des revendications 1 à 5, dans lequel le bras d'échantillon (110) comprend en outre une fibre de détection (117) adaptée pour recevoir l'image du point illuminé au niveau du plan de détection et guider de la lumière vers le photodétecteur et, plus préférablement un actionneur (170) conçu pour translater la fibre de détection (117) de manière latérale sur l'ensemble de l'image du point illuminé.

7. Appareil optique selon l'une quelconque des revendications 4 à 6, dans lequel l'unité de calcul (140, 1040) est configurée pour générer une image de volume de l'échantillon, de préférence un œil complet (200, 1200) et des fonctions d'étalement du point, PSF, et pour déterminer une erreur de front d'onde à l'aide d'un algorithme d'optique adaptative numérique, DAO, de préférence un algorithme d'optique adaptative numérique basé sur un cisaillement latéral numérique, DLS-DAO.

8. Appareil optique selon la revendication 7, dans lequel l'unité de calcul (140) est configurée pour :
obtenir des données volumétriques de tomographie par cohérence optique, données OCT, de balayages PSF de l'œil (200),
extraire un champ PSF en face au niveau d'une couche rétinienne de l'œil (200) après un traitement de données basé sur OCT,
dériver une distance de défocalisation dans un espace d'image à partir d'un décalage dans le plan d'image au niveau duquel une image du point lumineux est la mieux focalisée lorsque la longueur focale de l'optique à focalisation accordable (112) est modifiée,
calculer la FFT 2D du champ PSF et ajouter une phase de défocalisation correspondant à la distance défocalisée dérivée à la phase du champ de Fourier calculé,
propager de manière numérique et en ondes le champ résultant au niveau du plan de Fourier jusqu'à l'emplacement d'image de la pupille (201) de l'œil (200), et
reconstruire l'erreur de phase ou de front d'onde à l'aide de l'algorithme d'optique adaptative numérique, DAO, de préférence de l'algorithme DSL-DAO, à partir du champ calculé au niveau d'un plan de pupille (201) de l'œil (200).

9. Appareil optique selon la revendication 7, dans lequel l'unité de calcul (140) est configurée pour :
obtenir des données volumétriques de tomographie par cohérence optique, données OCT, de balayages PSF de l'œil (200),
extraire un champ PSF en face au niveau d'une couche rétinienne de l'œil après un traitement de données basé sur OCT,
calculer la FFT 2D du champ PSF,
propager de manière numérique et en ondes le champ résultant au niveau du plan de Fourier jusqu'à l'emplacement d'image de la pupille (201) de l'œil (200),
dériver une distance de défocalisation d'un plan focal de l'œil (200) par rapport à la rétine, à partir d'un changement de focalisation de l'optique à focalisation accordable (112),
ajouter une phase de défocalisation correspondant à la distance défocalisée dérivée à la phase du champ de pupille calculé, et
reconstruire l'erreur de phase ou de front d'onde à l'aide de l'algorithme d'optique adaptative numérique, DAO, de préférence de l'algorithme DSL-DAO, à partir du champ calculé au niveau d'un plan de pupille (201) de l'œil (200).

10. Appareil optique selon la revendication 7, dans lequel l'unité de calcul (140) est configurée pour :
obtenir des données volumétriques de tomographie par cohérence optique, données OCT, de balayages PSF de l'œil (200) dans un état aphaque,
extraire un champ PSF en face au niveau d'une couche rétinienne après un traitement de données basé sur OCT,
dériver une distance de défocalisation d'un plan focal de l'œil aphaque (200) par rapport à la rétine, à partir d'un changement de focalisation de l'optique à focalisation accordable (112),
propager de manière numérique et en ondes le champ PSF vers un plan d'emplacement estimé de lentille intraoculaire, LIO (209) à l'intérieur de l'œil (200) en tenant compte de la distance de défocalisation, et
reconstruire l'erreur de phase ou de front d'onde à l'aide de l'algorithme d'optique adaptative numérique, DAO, de préférence de l'algorithme DSL-DAO, à partir du champ calculé au niveau du plan d'emplacement LIO (209) à l'intérieur de l'œil (200).

11. Appareil optique selon la revendication 10, dans lequel l'unité de calcul (140) est configurée pour déterminer au moins l'une parmi une valeur sphérique, une valeur cylindrique et une valeur d'axe cylindrique à partir de l'erreur de phase reconstruite et pour utiliser les valeurs déterminées pour sélectionner une LIO (180) ou pour concevoir une LIO personnalisée (180) ayant une puissance sphérique et cylindrique et un axe cylindrique optimaux pour corriger l'erreur de phase ou de front d'onde de l'œil (200).

12. Appareil optique selon la revendication 10 ou 11, dans lequel l'unité de calcul (140) est configurée pour évaluer des performances d'une LIO (180), comprenant de préférence :
la multiplication d'un champ au niveau du plan d'emplacement LIO (209) par une exponentielle complexe d'une phase connue d'une LIO sélectionnée (180),
le recalcul du champ au niveau du plan rétinien (202) à l'aide d'un algorithme de propagation numérique des ondes, et
le calcul d'une taille de point et d'une fonction de transfert de module, MTF, pour quantifier des performances visuelles.

13. Appareil optique selon la revendication 7, dans lequel l'unité de calcul (140) est configurée pour :
obtenir des données volumétriques de tomographie par cohérence optique, données OCT, de balayages PSF de l'œil (200) dans un état phaque,
extraire un champ PSF en face au niveau d'une couche rétinienne après un traitement de données basé sur OCT,
dériver une distance de défocalisation d'un plan focal de l'œil phaque (200) par rapport à la rétine, à partir d'un changement de focalisation de l'optique à focalisation accordable (112),
propager de manière numérique et en ondes le champ PSF jusqu'à un plan au niveau de la dernière surface d'un cristallin compte tenu d'un milieu vitreux à l'intérieur de l'œil (200) et en tenant compte d'une distance de défocalisation,
multiplier le champ calculé par un conjugué complexe d'une fonction de transmission du cristallin afin d'annuler son effet de réfraction,
propager de manière numérique et en ondes le champ résultant jusqu'à un emplacement estimé de lentille intraoculaire, LIO (209), et
reconstruire l'erreur de phase ou de front d'onde à l'aide de l'algorithme d'optique adaptative numérique, DAO, de préférence de l'algorithme DSL-DAO, à partir du champ calculé au niveau d'un plan d'emplacement LIO (209) à l'intérieur de l'œil.

14. Appareil optique selon la revendication 13, dans lequel l'unité de calcul (140) est configurée pour déterminer au moins l'une parmi une valeur sphérique, une valeur cylindrique et une valeur d'axe cylindrique à partir de l'erreur de phase reconstruite et pour utiliser les valeurs déterminées pour sélectionner une LIO (180) ou pour concevoir une LIO personnalisée (180) ayant une puissance sphérique et cylindrique et un axe cylindrique optimaux pour corriger l'erreur de phase ou de front d'onde de l'œil (200).

15. Appareil optique selon la revendication 13, dans lequel l'unité de calcul (140) est configurée pour évaluer des performances LIO, comprenant de préférence :
la multiplication d'un champ au niveau du plan d'emplacement LIO (209) par la fonction de transmission d'une LIO sélectionnée (180),
le recalcul du champ au niveau du plan rétinien (202) à l'aide d'un algorithme numérique de propagation d'ondes, et
le calcul d'une taille de point et d'une fonction de transfert de module, MTF, pour quantifier des performances visuelles.

16. Appareil optique selon l'une quelconque des revendications 1 à 15, dans lequel l'unité de calcul (140, 1040) est configurée pour :
fournir des PSF correspondant à la couche rétinienne et à l'erreur de front d'onde d'un œil (200, 1200),
déterminer une puissance et un axe cylindriques d'une lentille intraoculaire, LIO, torique corrigeant l'astigmatisme ou l'erreur cylindrique,
déterminer une erreur cylindrique résiduelle après l'implantation de la LIO torique dans un œil (200, 1200) pendant la chirurgie d'implantation de LIO à partir d'une métrique de qualité sur la base d'une taille de point du profil PSF,
déterminer un angle selon lequel la LIO torique doit être tournée pour obtenir un alignement d'axe optimal afin d'annuler l'erreur cylindrique résiduelle, et enfin, confirmer la précision de l'alignement d'axe de la LIO torique sur la base d'une métrique de qualité sur la base d'une taille de point du profil PSF.
